(19) Europäisches Patentamt
European Patent Office
Office européen des brevets

(11) EP 1 063 254 A1

(12) **EUROPÄISCHE PATENTANMELDUNG**

(43) Veröffentlichungstag:
**27.12.2000 Patentblatt 2000/52**

(21) Anmeldenummer: **99112260.7**

(22) Anmeldetag: **25.06.1999**

(51) Int Cl.[7]: **C08G 65/329**, C08G 65/333, A61K 48/00, C12N 15/87, A61K 47/48

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(71) Anmelder: **Technische Uni München, Klinikum rechts der Isar, Inst. für Experiment. Onkoligie und Therapieforschung
81675 München (DE)**

(72) Erfinder:
- **Plank, Christian Dr.
82229 Seefeld (DE)**
- **Finsinger, Dirk Dr.
81245 München (DE)**

(74) Vertreter: **Laudien, Dieter, Dr.
Boehringer Ingelheim GmbH
Abteilung Patente,
Postfach 200
55216 Ingelheim (DE)**

(54) **Copolymere für den Transport von Nukleinsäuren in die Zelle**

(57) Copolymere aus einem amphiphilen Polymer, vorzugsweise Polyethylenglykol, und einem geladenen Effektormolekül, insbesondere einem Peptid oder Peptidderivat.

Nukleinsäurekomplexe, in denen Nukleinsäure mit einem Polykation kondensiert und die an der Oberfläche das geladene Copolymer gebunden enthalten, als nichtvirale Vektoren für die Gentherapie.

**Fig. 8**

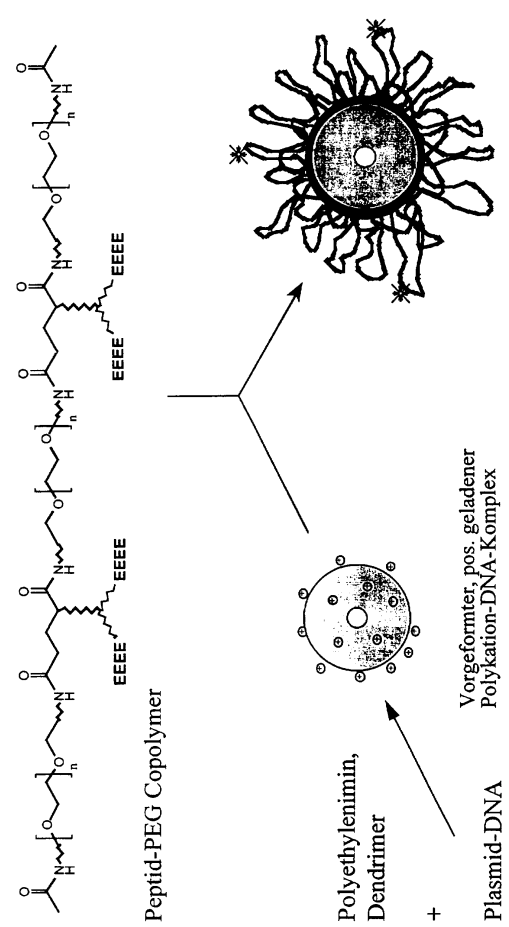


EP 1 063 254 A1

## Beschreibung

Die Erfindung bezieht sich auf das Gebiet des Gentransfers, insbesondere auf nichtvirale Vektoren.

Die Verfügbarkeit stabiler, effizienter Genvektoren ist Voraussetzung für die klinische Umsetzbarkeit gentherapeutischer Strategien. Allerdings sind die meisten bekannten Gentransfer-Vehikel bei der systemischen Anwendung mit dem Ziel der somatischen Gentherapie noch mit Problemen behaftet.

Für effizienten Gentransfer in vivo sind grundsätzlich folgende zwei Transportprobleme zu lösen: 1) Transfer des zu transferierenden Agens (z.B. Plasmid-DNA, Oligonukleotide) von der Applikationsstelle im Organismus zur Zielzelle (der extrazelluläre Aspekt) und 2) Transfer des zu transferierenden Agens von der Zelloberfläche in das Zytoplasma bzw. den Zellkern (der zelluläre Aspekt). Eine wesentliche Voraussetzung für den rezeptorvermittelten Gentransfer ist die Kompaktierung von DNA zu Partikeln von Virusgröße und Freisetzung der DNA aus internen Vesikeln nach endozytotischer Aufnahme in die Zellen. Diese Voraussetzung wird durch Kompaktierung von DNA mit bestimmten kationischen Polymeren erfüllt, deren chemische Beschaffenheit die Freisetzung von DNA-Komplexen aus internen Vesikeln (Endosomen, Lysosomen) nach endzytotischer Aufnahme in Zellen gewährleistet (Boussif et al., 1995; Ferrari et al., 1997; Haensler & Szoka, 1993; Tang et al., 1996). Solch ein Effekt wird auch durch den Einbau von pH-abhängigen membranzerstörenden Peptiden in DNA-Komplexe erzielt (Plank et al., 1994; WO 93/07283). Bei geeigneter Zusammensetzung der DNA-Komplexe kann eine spezifische Aufnahme und effizienter Gentransfer in Zellen durch Rezeptor-Liganden-Wechselwirkung erzielt werden (Kircheis et al., 1997, Zanta et al., 1997). Besonders geeignet für den rezeptorvermittelten Gentransfer sind auch Komplexe von DNA mit kationischen Peptiden (Gottschalk et al., 1996; Wadhwa et al., 1997; Plank et al., 1999).

Die klinische Umsetzung der vielversprechenden Forschungsergebnisse, die im Gentransfer mit nichtviralen Vektoren erzielt werden, wird unter anderem dadurch erschwert, daß der extrazelluläre Aspekt des Transportproblems nur unvollständig gelöst ist. Eine der Ursachen für dieses Problem ist die chemisch-physikalische Beschaffenheit der nichtviralen Gentransfervektoren, aufgrund derer sie bei systemischer Anwendung starke Wechselwirkungen mit Blut- und Gewebekomponenten eingehen (z.B. durch Opsonisierung, das Anheften von Serumprotein), wodurch insbesondere der auf bestimmte Zielzellen ausgerichtete rezeptorvermittelte Gentransfer eingeschränkt wird. Es wurde gezeigt, daß die Oberflächenmodifikation von DNA-Komplexen mit Poly(ethylenglykol) deren Blutprotein-bindende Eigenschaften entscheidend vermindert (Plank et al., 1996; Ogris, 1998; WO 98/59064). Eine weitere Einschränkung des Einsatzes nichtviraler Vektoren besteht in der unzureichenden Löslichkeit (bzw. Stabilität) von DNA-Komplexen in vivo. Mit den bekannten Methoden war es bisher nicht möglich, DNA in ausreichend hohen Konzentrationen für intravenöse Anwendungen (z.B. im Bereich von 1 mg/ml) mit einem Polykation zu komplexieren, weil die DNA-Komplexe unter physiologischen Salzkonzentrationen aggregieren und aus der Lösung ausfallen.

Ähnliche Probleme treten auch bei der Applikation niedermolekularer chemischer Verbindungen auf. Auf dem Gebiet der "klassischen" Arzneimittel werden biologisch abbaubare synthetische Polymere dazu verwendet, Pharmazeutika in solch einer Form zu verpacken, die erhöhte Verweildauer im Organismus gewährleistet und zur gewünschten biologischen Verfügbarkeit im Zielorgan führt ("controlled release"). Dafür wird die Oberflächenmodifikation von kolloidalen Partikeln mit Polyethylenglykol derart gestaltet, daß die unerwünschte Opsonisierung unterdrückt wird. Über die Synthese und Charakterisierung biologisch abbaubarer Polymere für den Einsatz bei einer Vielzahl medizinischer Anwendungen existiert umfangreiche Literatur (Coombes et al., 1997). Je nach Substanz und Applikation werden die chemischen Bindungen im Polymerrückgrat variiert. Durch geeignete Positionierung von Ester-, Amid-, Peptid- oder Urethanbindungen kann die gewünschte Labilität in physiologischem Milieu erreicht werden, wobei die Sensitivität gegenüber den Angriff von Enzymen gezielt variiert wird. Für eine schnelle und effiziente Synthese von biologisch wirksamen Substanzen haben sich kombinatorische Syntheseprinzipien bewährt (Balkenhohl et al., 1996). Durch systematische Variation weniger Parameter gelangt man zu einer großen Anzahl von Verbindungen, welche die gewünschte Grundstruktur aufweisen (Brocchini et al., 1997). Mit einem geeigneten, aussagekräftigen biologischen Selektionssystem können aus diesem Pool von Verbindungen diejenigen herausgesucht werden, welche die gewünschten Eigenschaften zeigen.

In dem US-Patent Nr. 5,455,027 werden Polymere beschrieben, die aus alternierenden Einheiten eines Polyalkylenoxids und eines funktionalisierten Alkans bestehen, wobei an die funktionelle Seitengruppe des Alkans ein pharmokologischer Wirkstoff kovalent gekoppelt ist.

Im Laufe der letzten Jahre haben sich bei der Anwendung nichtviraler Gentransfersysteme folgende wesentliche Erkenntnisse herauskristallisiert:

a)Komplexe aus Plasmid-DNA und kationischen Polymeren eignen sich zum Gentransfer in vitro und in vivo, wobei Komplexen mit Polymeren mit sekundären und tertiären Aminogruppen auch eine inhärente endosomolytische Aktivität zugesprochen werden kann, die zu effizientem Gentransfer führt (Boussif et al., 1995; Tang et al., 1996).
b) Verzweigte kationische Peptide eignen sich ab einer gewissen Kettenlänge des kationischen Anteils zur effizienten Bindung an DNA und zur Bildung von partikulären DNA-Komplexen (Plank et al., 1999).

c) Polykation-DNA-Komplexe gehen starke Wechselwirkungen mit Blutkomponenten ein und aktivieren das Komplementsystem (Plank et al., 1996).

d) Starke Wechselwirkungen von partikulären Strukturen mit Blutkomponenten können durch Modifikation mit Polyethylenglykol vermindert oder verhindert werden; dies gilt auch für Polykation-DNA-Komplexe (Plank et al., 1996; Ogris et al., 1999).

Der vorliegenden Erfindung lag die Aufgabe zugrunde, ein neues, verbessertes nichtvirales Gentransfersystem auf der Grundlage von Nukleinsäure-Polykation-Komplexen bereitzustellen.

Bei der Lösung der im Rahmen der vorliegenden Erfindung zugrundeliegenden Aufgabe wurde von der Überlegung ausgegangen, die Nukleinsäure bzw. Nukleinsäure-Komplexe mit einem geladenen Polymer zu umhüllen, welches die Komplexe physikalisch stabilisiert und sie vor Opsonisierung schützt.

Die vorliegende Erfindung betrifft in einem ersten Aspekt ein geladenes Copolymer der allgemeinen Formel I

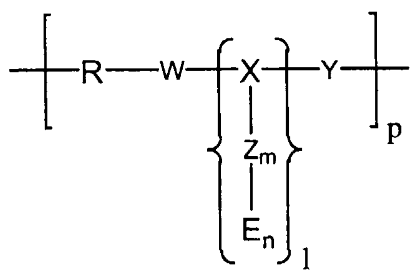

wobei R amphiphiles Polymer oder ein homo- oder hetero-bifunktionelles Derivat davon ist,
und worin X

i) eine Aminosäure oder ein Aminosäurederivat, ein Peptid oder ein Peptidderivat oder ein Spermin- oder Spermidinderivat ist; oder

ii) worin X

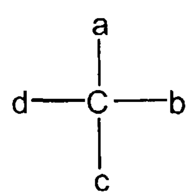

ist, wobei
a H oder, gegebenenfalls halogen- oder dialkylamino-substituiertes, $C_1$-$C_6$-Alkyl bedeutet;
und wobei
b, c und d gleiches oder verschiedenes, gegebenenfalls halogen- oder dialkylamino-substituiertes $C_1$-$C_6$-Alkylen bedeutet; oder

iii) worin X

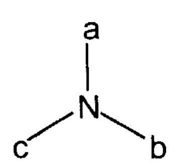

ist, wobei
a H oder, gegebenenfalls halogen- oder dialkylamino-substituiertes, $C_1$-$C_6$-Alkyl bedeutet, und wobei
b und c gleiches oder verschiedenes, gegebenenfalls halogen- oder dialkylamino-substituiertes $C_1$-$C_6$-Alkylen bedeuten; oder

iv) worin X
eine substituierte aromatische Verbindung mit drei funktionellen Gruppierungen $W_1Y_1Z_1$ ist, wobei W, Y und Z die unten angegebenen Bedeutungen haben;
wobei
W, Y oder Z gleiche oder verschiedene Reste CO, NH, O oder S oder eine zur Reaktion mit SH, OH, NH oder $NH_2$ befähigte Linkergruppierung sind;
und wobei das Effektormolekül E
ein kationisches oder anionisches Peptid oder Peptidderivat oder ein Spermin- oder Spermidinderivat oder ein Glykosaminoglycan oder ein nichtpeptidisches Oligo/Poly-kation oder -anion; worin
m und n unabhängig voneinander 0, 1 oder 2 sind; worin
p vorzugsweise 3 bis 20; und worin
l 1 bis 5, vorzugsweise 1 ist.

Für den Fall, daß l >1 ist, kann die Einheit X-$Z_m$-$E_n$ gleich oder verschieden sein.
Aromat bedeutet im Sinne der Erfindung einen monocyclischen oder bicyclischen aromatischen Kohlenwasserstoffrest mit 6 bis 10 Ringatomen, der - neben den eingangs genannten Substituenten - gegebenenfalls unabhängig mit einem oder mehreren weiteren Substituenten, vorzugsweise mit einem , zwei oder drei Substituenten ausgewählt aus der Gruppe $C_1$-$C_6$-Alkyl, -O-( $C_1$-$C_6$-Alkyl), Halogen - vorzugsweise Fluor, Chlor oder Brom - Cyano, Nitro, Amino, mono-($C_1$-$C_6$-Alkyl)amino, di-($C_1$-$C_6$-Alkyl)amino substituiert sein kann. Bevorzugt ist der Phenylrest.
Aromat im Sinne der vorliegenden Erfindung kann auch für einen Heterorarylrest, d.h.: einen monocyclischen oder bicyclischen aromatischen Kohlenwasserstoffrest mit 5 bis 10 Ringatomen stehen , der ein, zwei oder drei Ringatome ausgewählt aus der Gruppe N, O oder S unabhängig von einander enthält, wobei die restlichen Kringatome C bedeuten.
Alkylamino, bzw. Dialkylamino steht - sofern nicht anders angegeben für eine Aminogruppe, die mit einem oder zwei $C_1$-$C_6$-Alkylresten substituiert ist, wobei - im Fall von zwei Alkylresten - die beiden Alkylgruppen auch einen Ring bilden können.
$C_1$-$C_6$-Alkyl steht im allgemeinen - sofern nicht anders angegeben - für einen verzweigten oder unverzweigten Kohlenwasserstoffrest mit 1 bis 6 Kohlenstoffatom(en), der gegebenenfalls mit einem oder mehreren Halogenatom (en) - vorzugsweise Fluor - substituiert sein kann, die untereinander gleich oder verschieden sein können. Als Beispiele seien folgende Kohlenwasserstoffreste genannt:
Methyl, Ethyl, Propyl, 1-Methylethyl (Isopropyl), n-Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2,-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.
Bevorzugt sind - sofern nicht anders angegeben - Niederalkylreste mit 1 bis 4 Kohlenstoffatomen, wie Methyl, Ethyl, Propyl, *iso*-Propyl, n-Butyl, 1-Methylpropyl, 2-Methylpropyl oder 1,1-Dimethylethyl.
Entsprechend bedeutet Alkylen eine verzweigte oder unverzweigte zweibindige Kohlenwasserstoffbrücke mit 1 bis 6 Kohlenstoffatomen, die gegebenenfalls mit einem oder mehreren Halogenatom(en) - vorzugsweise Fluor - substituiert sein kann, die untereinander gleich oder verschieden sein können.
Das amphiphile Polymer R ist bevorzugt ein Polyalkylenoxid, Polyvinylpyrollidon, Polyacrylamid, Polyvinylalkohol, oder ein Copolymer dieser Polymere.
Beispiele für geeignete Polyalkylenoxide sind Polyethylenglykole (PEG), Polypropylenglykole, Polyisopropylenglykole, Polylbutylenglykole.
Bevorzugt im Rahmen der vorliegenden Erfindung sind Polyalkylenoxide, insbesondere PEG.
Das Polyalkylenoxid kann im Copolymer als solches vorliegen, oder als Thio-, Carboxy- oder Aminoderivat.
Das Polymer R weist bevorzugt ein Molekulargewicht von 500- 10 000 auf, vorzugsweise 1000 - 10 000.
Für den Fall i), in dem X eine Aminosäure ist, kann für die Synthese des Copolymeren eine Aminosäure mit drei funktionellen Gruppen eingesetzt werden, wobei zwei dieser Gruppen zur Copolymerisation mit dem Polymer und eine zur Ankoppelung des Effektorsmoleküls E befähigt sind; in diesem Fall kann Z entfallen. Bevorzugt sind die natürlichen Aminosäuren Glutaminsäure, Asparaginsäure, Lysin, Ornithin und Tyrosin. Prinzipiell können statt der natürlichen Aminosäuren auch synthetische verwendet werden (z.B. entsprechende Spermin- und Spermidinderivate).
Im Fall i) kann ein für die Synthese auch ein Aminosäurederivat verwendet werden, das zwei funktionelle

Gruppen zur Copolymerisation mit dem Polymeren enthält und durch Modifikation einer Aminosäure (Glutaminsäure, Asparaginsäure, Lysin oder Ornithin) mit einer Linkergruppierung zur Ankoppelung des Effektormoleküls erhalten wurde, womit Z entfällt (m = 0); Beispiele für Linkergruppierungen sind Pyridylthiomercaptoalkylcarboxylate (siehe Fig. 1) oder Maleimidoalkancarboxylate.

Im Fall i) kann X auch ein Peptid(derivat) sein. Im Fall eines ungeladenen Peptids oder Peptidderivats ist daran E direkt oder über Z gekoppelt. Für den Fall, daß X ein positiv oder negativ geladenes Peptid oder Peptidderivat oder ein Spermin- oder Spermidinderivat ist, stellt X selbst das Effektormolekül dar (Z und E entfallen, m=n=0). Im einfachsten Fall besteht das Peptid in diesem Fall aus einer linearen Abfolge aus zwei oder mehreren identischen oder verschiedenen natürlichen oder synthetischen Aminosäuren dar, wobei die Aminosäuren so gewählt werden, daß das Peptid insgesamt entweder positiv oder negativ geladen ist. Alternativ kann das Peptid verzweigt sein. In diesen Fällen stellt das Peptid als solches den Effektor dar, Z und E entfallen (m = n = 0). Beispiele für einen Typ geeigneter verzweigter kationischer Peptide wurden von Plank et al., 1999, beschrieben.

Geeignete anionische Peptidderivate X weisen die allgemeine Formel $(\text{Peptid})_n$-B-Spacer-(Xaa) auf. Das Peptid ist eine Abfolge von Aminosäuren oder Aminosäurederivaten mit insgesamt negativer Ladung. Bevorzugt besteht das Peptid aus drei bis 30 Aminosäuren, vorzugsweise besteht es ausschließlich aus Glutaminsäure- und/oder Asparaginsäureresten. n stellt die Anzahl der Verzweigungen in Abhängigkeit der in B enthaltenen funktionellen Gruppen dar. B ist ein Verzweigungsmolekül, vorzugsweise Lysin oder ein Molekül des Typs X im Fall von ii) bis iv). Der Spacer ist ein Peptid, bestehend aus 2 bis 10 Aminosäuren oder eine organische Aminocarbonsäure mit 3 bis 9 Kohlenstoffatomen im Carbonsäuregerüst, z.B. 6-Aminohexansäure. Der Spacer dient zur räumlichen Trennung des geladenen Effektormoleküls vom Polymergerüst. Xaa ist bevorzugt eine trifunktionelle Aminosäure, insbesondere Glutaminsäure oder Asparaginsäure und kann im allgemeinen eine Verbindung des Typs X, Fall i) - iv) sein.

Alternativ kann X im Fall i) ein Peptidderivat sein, wobei die Modifikation des Peptids in einer geladenen Gruppierung besteht, die von einer Aminosäure verschieden ist; Beispiele für derartige Gruppierungen sind Sulfonsäuregruppierungen oder geladene Kohlenhydratreste, wie Neuraminsäuren, oder sulfatierte Glycosaminoglycane. Die Modifikation des Peptids kann nach Standardmethoden durchgeführt werden, entweder direkt im Zuge der Peptidsynthese oder, nachträglich am fertigen Peptid.

Das Effektormolekül E kann, wie X im Fall i), ein polykationisches oder polyanionisches Peptid oder Peptidderivat oder ein Spermin- oder Spermidinderivat sein. Im einfachsten Fall stellt das Peptid auch in diesem Fall eine lineare Abfolge aus zwei oder mehreren identischen oder verschiedenen natürlichen oder synthetischen Aminosäuren dar, wobei die Aminosäuren so gewählt werden, daß das Peptid insgesamt entweder positiv oder negativ geladen ist. Alternativ kann das Peptid verzweigt sein. Beispiele für geeignete verzweigte kationische Peptide wurden von Plank et al., 1999, beschrieben. Geeignete anionische Moleküle E weisen die allgemeine Formel $(\text{Peptid})_n$-B-Spacer-(Xbb) auf, wobei Xbb vorzugsweise eine Aminosäure mit einer reaktiven Gruppe ist, welche direkt oder über Z an X koppelbar ist.

Die Koppelung des Effektorpeptids E an Z oder direkt an X erfolgt über eine im Peptid von vornherein vorhandene oder nachträglich eingeführte reaktive Gruppe, z.B. eine Thiolgruppe (in einem Cystein oder durch Einführung einer Mercaptoalkansäuregruppe). Alternativ, in Abhängigkeit von Z, kann die Koppelung auch über bereits vorhandene oder nachträglich eingeführte Amino- oder Carbonsäuregruppen erfolgen.

Alternativ kann E, wie X im Fall i), ein Peptidderivat sein, wobei die Modifikation des Peptids in einer geladenen Gruppierung besteht, die von einer Aminosäure verschieden ist, Beispiele für derartige Gruppierungen sind Sulfonsäuregruppierungen oder geladene Kohlenhydratreste, wie Neuraminsäuren, oder sulfatierte Kohlenhydratreste. Die Koppelung an X erfolgt auch in diesem Fall direkt oder über Z.

Das Copolymer der allgemeinen Formel I

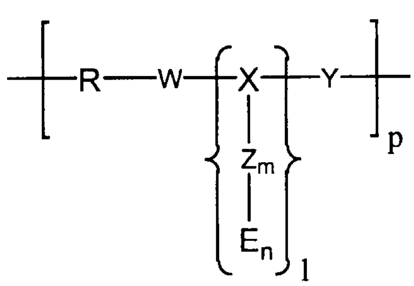

ist bevorzugt als streng alternierendes Block-Copolymer aufgebaut.

Gegebenenfalls ist das Copolymer mit einem zellulären Liganden für die Zielzelle (Rezeptorligand L) modifiziert. In diesem Fall ist die Mehrzahl der Linkerpositionen Z mit E besetzt, dazwischen ist an einzelne Positionen des Linkers Z an Stelle des kationischen bzw. anionischen Effektors E ein zellulärer Ligand gekoppelt. Alternativ liegt der Ligand an einzelne Positionen des Effektormoleküls E gekoppelt vor. Bevorzugt beträgt das Verhältnis E:L ca. 10:1 bis 4:1. Der Rezeptorligand kann biologischen Ursprungs sein (z.B. Transferrin, Antikörper, Kohlenhydratreste) oder synthetisch (z.B. RGD-Peptide, synthetische Peptide, Derivate von synthetischen Peptiden); Beispiele für geeignete Liganden sind in der WO 93/07283 angegeben.

Die erfindungsgemäßen Copolymeren können nach folgendem Verfahrensschema hergestellt werden:

Der Copolymerisationspartner X bzw. X-$Z_m$-$E_n$ wird, sofern er ein Peptid oder Peptidanalog ist, nach Standardmethoden, z.B. an der festen Phase (solid phase peptide synthesis, SPPS) nach Fmoc-Protokoll synthetisiert (Fields et al., 1990). Die Aktivierung der Aminosäurederivate erfolgt dabei mit TBTU/HOBt oder mit HBTU/HOBt (Fields et al., 1991). Verwendet werden für die ionischen Aminosäurepositionen folgende Derivate in ihrer N-terminal Fmoc-geschützten Form:

(a) kationische Seitenketten: R(Pbf), K(Boc,Trt), Ornithin(Boc), Caboxyspermin oder -spermidin (Boc).

(b) anionische Seitenketten: D(O-tert.Bu), E(O-tert.Bu).

Für die Verzweigungsstelle B des Moleküls $(Peptid)_n$-B-Spacer-(Xaa) oder $(Peptid)_n$-B-Spacer-(Xbb) kommt Fmoc-K(Fmoc)-OH zum Einsatz. Die Peptide werden mit TFA/DCM vom Harz gespalten.

Wenn der Polymerisationspartner X ein Peptid der allgemeinen Struktur $(Peptid)_n$-B-Spacer-(Xaa) in der nachfolgenden Copolymerisation ist, so wird an der Position Xaa Glutaminsäure oder Asparaginsäure verwendet, die an einer Carboxyl-Position mit einer Benzylschutzgruppe versehen ist. Diese wird selektiv durch Hydrogenolyse (Felix et al., 1978) entfernt. Die N-terminal gelegenen Aminosäurepositionen der Peptidkette werden mit Boc-geschützten Aminosäuren belegt, um die Schutzgruppenabspaltung nach Copolymerisation des Peptids mit PEG in einem Schritt durchführen zu können.

Ist der Polymerisationspartner X ein Aminosäurederivat, das eine Linkergruppierung enthält (z.B. 3-Mercaptopropionsäure, 6-Aminohexansäure), so kann dieses in Flüssigphase nach klassischen Methoden der Peptidchemie erhalten werden. Mercaptopropionsäure wird mit 2,2'-Dithiodipyridin umgesetzt und chromatographisch gereinigt. Das Reaktionsprodukt wird mit Carboxyl-geschützter Glutaminsäure (O-t.butyl) unter HOBt/EDC-Aktivierung umgesetzt (siehe Fig. 1). Analog wird 6-Fmoc-Aminohexansäure umgesetzt. Die Carboxylschutzgruppen werden in TFA/DCM entfernt, das resultierende Glutaminsäurederivat wird mittels chromatographischer Methoden gereinigt.

Die Herstellung der Copolymeren ist nach folgenden Prinzipien möglich und wird anhand eines PEG-Peptid-Copolymeren veranschaulicht:

(1) poly(PEG -O- OC-) Matrix ("Polyester"):
Die Copolymerisation der ionischen, partiell seitenkettengeschützten Peptid-Dicarbonsäuren bzw. Glutamin- oder Asparaginsäurederivate mit PEG-Macromonomeren in definierten Molmassenbereichen (MW 400-20000 in Handel erhältlich, z.B. Fluka) liefert eine Matrix auf PEG-Esterbasis. Diese stellt ein in physiologischer Umgebung hydrolyselabiles System dar (Ulbrich et al., 1985).
Die p(PEG-Peptid)-Copolymere werden dabei nach etablierten Methoden, z.B. mit Dicyclohexylcarbodiimid/DMAP, vorzugsweise in streng alternierender Folge aufgebaut (Zalipsky et al., 1984; Nathan, A., 1992). Das PEG-Macromonomer wird zusammen mit einem seitenkettengeschützten Peptid bzw. Glutamin- oder Asparaginsäurederivat in Dichlormethanlösung mit DCC / DMAP versetzt. Nach Abtrennung des entstandenen Harnstoffderivates kann das Polymer durch Fällung mit kaltem Äther erhalten werden. Die Abspaltung der noch verbliebenen Seitenkettenschutzgruppen gelingt mit TFA in Dichlormethan (unter diesen Bedingungen ist auch die PEG-Ester-Bindung stabil (Zalipsky et al., 1984)). Das ionische Polymer wird durch Ausfällen und abschließenden Chromatographieschritt erhalten. Die Reaktionsführung ermöglicht eine Kontrolle des Polymerisationsgrades und des Ladungsanteils pro PEG-Einheit im Polymer.

(2) poly(PEG -HN- OC-) Matrix ("Polyamid")
Alternativ zum Polyester kann eine amidische Polymermatrix aufgebaut werden, falls, bei Verwendung eines Copolymer-DNA-Komplexes in einer gentherapeutischen Anwendung, eine zu schnelle Hydrolysierbarkeit und daher eine zu hohe Instabilität bei systemischer Applikation zu erwarten ist. In diesem Fall werden Diamino-PEG-Derivaten anstelle der der PEG-Macromonomere verwendet, die in Analogie zur oben beschriebenen Synthese mit den ionischen Peptiden bzw. Glutamin- oder Asparaginsäurederivaten copolymerisiert werden. Bei dieser Synthese wird ein hydrolysestabiles Amidgerüst erhalten. Diamino-modifizierte Polyethylenglykole sind als Grundstoffe in definierten Molmassenbereichen zwischen 500 und 20000 im Handel erhältlich (z.B. Fluka). Die verbliebenen säurelabilen Seitenkettenschutzgruppen der Peptidkomponenten werden, z.B. mit mit TFA/DCM abgespalten, und

die Polymere werden mittels chromatographischer Methoden gereinigt. Copolymere aus Glutamin- oder Asparaginsäurederivaten werden in einem weiteren Schritt mit anionischen oder kationischen Peptiden umgesetzt, die eine geeignete reaktive Gruppe enthalten. Z.B. werden Copolymere der 3-(2'-thio-pyridyl)-mercaptopropionyl-glutaminsäure mit Peptiden umgesetzt, die eine freie Cystein-Thiolgruppe enthalten. Von Copolymeren, die aus 6-Fmoc-aminohexanoyl-glutaminsäure hervorgegangen sind, wird unter basischen Bedingungen die Fmoc-Schutzgruppe entfernt. Das Produkt wird mit einem carboxylaktivierten, geschützten Peptid umgesetzt. Die Peptidschutzgruppen (t-Boc bzw. O-t.butyl) werden in DCM/TFA entfernt, das Endprodukt wird chromatographisch gereinigt. Alternativ kann die Aminogruppe von Ahx mit bifunktionellen Linkern derivatisiert werden und anschließend mit einem Peptid umgesetzt werden.

Die Kopplung des Liganden L kann direkt durch Aktivierung von Carboxylgruppen am Effektor E (bevorzugt bei anionischen Copolymeren) bzw. am Liganden erfolgen oder über Zwischenschaltung bifunktioneller Linker wie Succinimidyl-pyridyl-dithioproprionat (SPDP; Pierce oder Sigma) und ähnlicher Verbindungen. Die Reinigung des Reaktionsprodukts kann durch Gelfiltration und Ionenaustauschchromatographie durchgeführt werden.

Die Umsetzung dieses Copolymerisationsansatzes kann auch nach kombinatorischen Prinzipien erfolgen. Als selektierbare Variable treten dabei vor allem der Typ und das Molekulargewicht (Polymerisationsgrad) des Polymeren R, die Identität des Polymerisationspartners $X\text{-}Z_m\text{-}E_n$ bzw. des Effektormoleküls E (z.B. eine Serie von anionischen Peptiden mit einer zunehmenden Anzahl von Glutaminsäuren) und der Gesamtpolymerisationsgrad p auf.

Durch Variation von Molmassen der PEG-Macromonomere, der Art der verwendeten ionischen Spezies sowie deren Anteil am Copolymer und des Polymerisationsgrades der Polymermatrix ist ein Mehrparametersystem geschaffen, welches die schnelle Parallelkonstruktion einer homologen Reihe unterschiedlicher Copolymerer und in der Folge, nach Komplexierung mit der Nukleinsäure, verschiedener nichtviraler Vektoren ermöglicht. Das Synthesekonzept wird hierfür im Maßstab einer Zellkulturplatte (z.B. 96 wells per plate) umgesetzt. Dazu wird die chemische Synthese an den benötigten Mikromaßstab angepaßt (Reaktionsvolumina im Bereich von 500 µl). Dies ermöglicht die direkte Übertragung der parallel synthetisierten Polymere in den biologischen Assay und trägt so zu einem schnellen "screening" einer Vielzahl von Systemen und zum schnellen Auffinden geeigneter Verbindungen beitragen. Für die Durchführung des biologischen Auswahlverfahrens im Hinblick auf die bevorzugte Verwendung der erfindungsgemäßen Copolymeren für den Gentransfer werden die Copolymere z.B. mit DNA-Komplexen versetzt und dann Tests unterzogen, die eine Beurteilung der Polymereigenschaften bezüglich der beabsichtigten Anwendung (z.B. Gentransfer) zulassen. Ebensolche Auswahlverfahren können für Copolymerumhüllte Nanopartikel angewandt werden. Solche Screening- und Selektionsverfahren können z.B. Komplementaktivierungstests im 96-Well-Plate-Format dienen (Plank et al., 1996), turbidimetrische Messungen der durch Serumalbumin oder Salz induzierten Aggregation im gleichen Format oder in vitro Gentransferstudien im gleichen Format (Plank et al., 1999) oder fluoreszenzoptische Verfahren im gleichen Format sein.

Solche Untersuchungen geben z.B. Auskunft darüber, welche Copolymere aus einem kombinatorischen synthetischen Ansatz sich dazu eignen, die Oberfläche von DNA-Komplexen so zu modifizieren, daß deren Löslichkeit ausreichend für Gentransferapplikationen in vivo ist, deren Wechselwirkung mit Blut- und Gewebekomponenten eingeschränkt wird, sodaß deren Verweil- und Wirkdauer im Blutkreislauf ausreichend erhöht wird, um rezeptorvermittelten Gentransfer in Zielzellen zu ermöglichen.

Die erfindungsgemäßen Copolymere werden bevorzugt für den Transport von Nukleinsäuren in höhere eukaryotische Zellen verwendet.

Die vorliegende Erfindung betrifft somit in einem weiteren Aspekt Komplexe, enthaltend ein oder mehrere Nukleinsäuremoleküle und ein oder mehrere geladene Copolymere der allgemeinen Formel I.

Vorzugsweise ist das Nukleinsäuremolekül mit einem organischen Polykation oder einem kationischen Lipid kondensiert.

In einem weiteren Aspekt betrifft die Erfindung somit Komplexe aus Nukleinsäure und einem organischen Polykation oder einem kationischen Lipid, die dadurch gekennzeichnet sind, daß sie an ihrer Oberfläche ein geladenes Copolymer der allgemeinen Formel I über ionische Wechselwirkung gebunden haben.

Bei den in die Zelle zu transportierenden Nukleinsäuren kann es sich um DNAs oder RNAs handeln, wobei hinsichtlich der Nukleotidsequenz und Größe keine Beschränkungen bestehen. Die in den erfindungsgemäßen Komplexen enthaltene Nukleinsäure wird vor allem durch den in der Zelle zu erzielenden biologischen Effekt definiert, z. B. im Falle der Anwendung im Rahmen der Gentherapie durch das zur Expression zu bringende Gen bzw. den Genabschnitt, oder durch die beabsichtigte Substitution oder Reparatur eines defekten Gens oder einer beliebigen Zielsequenz (Yoon et al., 1996; Kren et al. 1998), oder durch die Zielsequenz eines zu inhibierenden Gens (z.B. im Fall der Anwendung von Antisenseoligoribonukleotiden oder Ribozymen). Vorzugsweise handelt es sich bei der in die Zelle zu transportierenden Nukleinsäure um Plasmid-DNA, die eine für ein therapeutisch wirksames Protein kodierende Sequenz enthält. Für die Anwendung im Rahmen einer Krebstherapie kodiert die Sequenz z.B. für ein oder mehrere Zytokine, wie Interleukin-2, IFN-α, IFn-γ, TNF-α, oder für ein Selbstmordgen, das in Kombination mit dem Substrat zur

Anwendung kommt. Für die Anwendung bei der sog. genetischen Tumorvakzinierung enthalten die Komplexe DNA, kodierend für ein oder mehrere Tumorantigene oder Fragmente davon, gegebenenfalls in Kombination mit DNA, kodierend für ein oder mehrere Zytokine. Weitere Beispiele für therapeutsich wirksame Nukleinsäuren sind in der WO 93/07283 angegeben.

Das erfindungsgemäße Copolymer hat die Eigenschaft, den Nukleinsäure-Polykation-Komplex sterisch zu stabilisieren und dessen unerwünschte Wechselwirkung mit Komponenten von Körperflüssigkeiten (z.B. mit Serumproteinen) zu vermindern oder zu unterdrücken.

Geeignete organische Polykationen für die Komplexierung von Nukleinsäure für den Transport in eukaryotische Zellen sind bekannt; aufgrund ihrer Wechselwirkung mit der negativ geladenen Nukleinsäure wird diese kompaktiert und in eine für die Aufnahme in die Zellen geeignete Form gebracht. Beispiele sind Polykationen, die für den rezeptorvermittelten Gentransfer verwendet wurden (EP 0388 758; WO 93/07283), wie homologe lineare kationische Polyaminosäuren (wie Polylysin, Polyarginin, Polyornithin) oder heterologe lineare gemischt kationisch-neutrale Polyaminosäuren (bestehend aus zwei oder mehreren kationischen und neutralen Aminosäuren), verzweigte und lineare kationische Peptide (Plank et al., 1999; Wadhwa et al. 1997), nichtpeptidische Polykationen (wie lineare oder verzweigte Polyethylenimine, Polypropylenimine), Dendrimere (sphäroidale Polykationen, die mit einem gut definierten Durchmesser und einer exakten Anzahl von endständigen Aminogruppen synthetisiert werden können; (Haensler und Szoka, 1993; Tang et al., 1996; WO 95/02397), kationische Kohlenhydrate, z.B. Chitosan (Erbacher et al. 1998). Die Polykationen können auch mit Lipiden modifiziert sein (Zhou et al. 1994; WO 97/25070).

Weitere geeignete Kationen sind kationische Lipide (Lee et al. 1997), die zum Teil im Handel erhältlich sind (z.B. Lipofectamin, Transfectam).

Im folgenden wird, wenn nicht anders angegeben, der Begriff "Polykation" stellvertretend sowohl für Polykationen als auch für kationische Lipide verwendet.

Im Rahmen der vorliegenden Erfindung bevorzugte Polykationen sind Polyethylenimine, Polylysin und Dendrimere, z.B. Polyamidoamindendrimere ("PAMAM"-Dendrimere).

Die Größe bzw. Ladung der Polykationen kann über einen weiten Bereich schwanken; sie wird so gewählt, daß der mit Nukleinsäure gebildete Komplex bei physiologischer Salzkonzentration nicht dissoziiert, was durch den Ethidiumbromidverdrängungsassay (Ethidiumbromide Displacement Assay) auf einfache Weise bestimmt werden kann (Plank et al, 1999). In einem weiteren Schritt wird eine definierte Menge an Nukleinsäure mit steigenden Mengen des ausgewählten Polykations inkubiert, der gebildete Komplex auf die zu transfizierenden Zellen aufgebracht und die Genexpression (im allgemeinen mit Hilfe eines Reportergenkonstrukts, z.B. Luciferase) nach Standardmethoden gemessen.

Der Aufbau der Nukleinsäure-Komplexe erfolgt über elektrostatische Wechselwirkungen. Die DNA kann im Verhältnis zum Polykation im Überschuß vorliegen, so daß derartige Komplexe eine negative Oberflächenladung aufweisen; im umgekehrten Fall, wenn das die Nukleinsäure kondensierende Polykation im Überschuß vorliegt, haben die Komplexe eine positive Oberflächenladung. Bevorzugt im Rahmen der vorliegenden Erfindung liegt das Polykation im Überschuß vor.

Bevorzugt wird das Verhältnis Polykation:Nukleinsäure im Falle eines positiven Ladungsüberschusses so eingestellt, daß das Zeta-Potential etwa +20 bis +50 mV beträgt, im Falle der Verwendung bestimmter Polykationen, z.B. Polylysin, kann es auch darüber liegen.

Im Falle eines negativen Ladungsüberschusses beträgt das Zetapotential etwa -50 bis -20mV.

Die Messung des Zeta-Potentials erfolgt nach etablierten Standardmethoden, wie z.B. von Erbacher et al. 1998, beschrieben.

Das Polykation ist gegebenfalls mit einem zellulären Liganden oder Antikörper konjugiert; geeignete Liganden sind in der WO 93/07283 beschrieben. Für den zielzellgerichteten Gentransfer im Rahmen einer Tumortherapie werden Liganden oder Antikörper für tumorzellassoziierte Rezeptoren (z.B. CD87; uPA-R) bevorzugt, die in der Lage sind, den Gentransfer in Tumorzellen zu erhöhen.

Bei der Herstellung der Komplexe wird die Nukleinsäure, im allgemeinen Plasmid-DNA, mit dem Polykation (ggf. derivatisiert mit einem Rezeptorliganden), das im Ladungsüberschuß vorliegt, inkubiert. Dabei bilden sich Partikel, die über rezeptorvermittelte Endzytose in Zellen aufgenommen werden können. Anschließend werden die Komplexe mit einem erfindungesgemäßen negativ geladenen Copolymer, vorzugsweise Polyethylenglykol-Copolymer, inkubiert. Der Effektor E im Copolymer ist bevorzugt ein polyanionisches Peptid. Alternativ wird zuerst das Copolymer, mit Nukleinsäure gemischt und dann mit Polykation inkubiert, oder, als dritte Variante, zuerst mit Polykation gemischt und dann mit Nukleinsäure inkubiert.

Alternativ wird die Nukleinsäure mit einem Polykation, das im elektrostatischen Unterschuß vorliegt, inkubiert und dann ein kationisches Copolymer zugefügt. Auch hier kann die Reihenfolge der Mischungsschritte variiert werden, wie oben für anionische Copolymere beschrieben. Die relativen Anteile der einzelnen Komponenten werden so gewählt, daß der resultierende DNA-Komplex ein schwach positives, neutrales oder schwach negatives Zeta-Potential aufweist (+10 mV bis -10 mV).

Im Fall der Verwendung positiv geladener Copolymerer können diese als alleinige Nukleinsäure-bindende und -kondensierende polykationische Moleküle verwendet werden; der Anteil eines Polykations oder kationischen Lipids kann somit entfallen. Die relativen Anteile der einzelnen Komponenten werden auch in diesem Fall so gewählt, daß der resultierende DNA-Komplex ein schwach positives, neutrales oder schwach negatives Zeta-Potential aufweist (+10 mV bis -10 mV).

In den Komplexen sind gegebenfalls Polykation und/oder Copolymer mit gleichen oder verschiedenen zellulären Liganden modifiziert.

Die erfindungsgemäßen Nukleinsäure-Komplexe, die durch das elektrostatisch gebundene Copolymer der allgemeinen Formel I in ihrer Größe stabilisiert und somit vor Aggregation geschützt sind, haben den Vorteil, in Lösung über längere Zeiträume (Wochen) lagerbar zu sein. Darüberhinaus haben sie den Vorteil, auf Grund der Schutzwirkung des gebundenen Copolymers eine verminderte bzw. keine Wechselwirkung mit Komponenten von Körperflüssigkeiten (z.B. mit Serumproteinen) einzugehen.

Die Erfindung betrifft in einem weiteren Aspekt eine pharmazeutische Zusammensetzung, enthaltend eine therapeutisch wirksame Nukleinsäure, das erfindungsgemäße Copolymer und gegebenenfalls ein organisches Polykation oder kationische Lipid.

Die erfindungsgemäße pharmazeutische Zusammensetzung liegt bevorzugt als Lyophilisat vor, gegebenfalls unter Zusatz von Zucker, wie Saccharose oder Dextrose, in einer Menge, die in der gebrauchsfertigen Lösung eine physiologische Konzentration ergibt. Die Zusammensetzung kann auch in Form eines Kryokonzentrats vorliegen.

Die erfindungsgemäße Zusammensetzung kann auch tiefgefroren (kryokonserviert) oder als gekühlte Lösung vorliegen.

In einem weiteren Aspekt dienen die erfindungsgemäßen, positiv oder negativ geladenen Copolymere dazu, kolloidale Partikel ("Nanopartikel"), wie sie für die Applikation klassischer Arzneimittel entwickelt werden, sterisch zu stabilisieren sowie deren unerwünschte Wechselwirkung mit Komponenten von Körperflüssigkeiten (z.B. mit Serumproteinen) zu vermindern oder zu unterdrücken. Weiters können die erfindungsgemäßen mit Rezeptorliganden modifizierten Copolymere dazu verwendet werden, solche Nanopartikel an ihrer Oberfläche mit Rezeptorliganden zu versehen, um Arzneimittel mit erhöhter Spezifität an Zielzellen zu transferieren ("Drug Targeting").

Figurenübersicht

Fig. 1: Herstellung der Copolymergerüste aus 3-(2'-thio-pyridyl)-mercaptopropionyl-glutaminsäure und O,O'-Bis (2-aminoethyl)poly(ethylenglykol) 6000 oder O,O'-Bis(2-aminoethyl)poly(ethylenglykol) 3400

Fig. 2: Ankoppelung von geladenen Peptiden an das Copolymergerüst

Fig. 3: Herstellung des Copolymergerüsts aus dem geschützten Peptid $E4E^{PROT}$ und O,O'-Bis(2-aminoethyl)poly (ethylenglykol) 6000

Fig. 4: Komplementaktivierungstests

Fig. 5: Erythrozyten-Lysetest

Fig. 6: Elektronenmikroskopischen Aufnahmen von PEI-DNA-Komplexen (N/P = 8) in Gegenwart des Copolymers P3YE5C

Fig. 7: Zetapotential von PEI- und DOTAP/Cholesterin-DNA-Komplexen in Abhängigkeit von der Menge zugesetzten Copolymers P3YE5C bzw. P6YE5C

Fig. 8: Herstellung DNA/Polykation/Copolymer-Komplexen

Fig. 9: Gentransfer in K562-Zellen mit PEI(25 kD)-DNA-Komplexen in Gegenwart und Abwesenheit des Copolymers P3YE5C

Fig. 10: Transfektion der Brustkrebszellinie MDA-MB435S mit Polylysin-DNA-Komplexen in Gegenwart und Abwesenheit des Hüllpolymers P3INF7

Fig. 11: Lipofection in NIH3T3-Zellen in Gegenwart und Abwesenheit des Copolymers P3YE5C

Fig. 12: Transfektion von HepG2-Zellen mit DOTAP/Cholesterin-DNA und PEI-DNA in Gegenwart und Abwesenheit von P6YE5C

Fig. 13: Intravenöser Gentransfer in vivo mittels DNA/Polykation-Komplexen mit Copolymerhülle

Beispiel 1

Herstellung von geladenen Copolymeren der allgemeinen Formel I

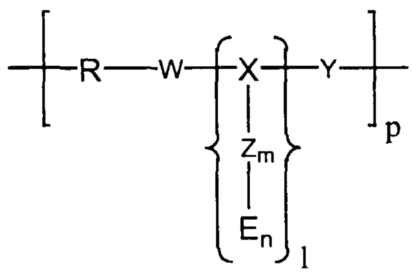

1.1. Herstellung der Copolymergerüste aus 3-(2'-thiopyridyl)-mercaptopropionyl-glutaminsäure und O,O'-Bis(2-aminoethyl)poly(ethylenglykol) 6000 oder O,O'-Bis(2-aminoethyl)poly(ethylenglykol) 3400 (Diamino-PEG-3400; Fluka)

In diesem Fall ist in der allgemeinen Formel I:

W=Y=NH;

X=3-mercaptopropionyl-glutaminsäure, das heißt, ein Aminosäurederivat gemäß Fall i), das durch Ankoppelung der Linkergruppierung 3-(2'-Thiopyridyl)-mercaptopropionsäure an Glutaminsäure erhalten wurde;
daher entfällt Z(m=0).

a) Umsetzung von 3-Mercaptopropionsäure mit 2,2'-Dithiodipyridin (1):

1 g DTDP (Fluka) wurde in 4 ml absolutem Ethanol (Merck) gelöst. Nach Zugabe von 100 µl Triethylamin (Aldrich) wurden 87 µl (lmmol) 3-Mercaptopropionsäure zugesetzt. Nach 1 h wurde das Reaktionsgemisch in Aliquots mittels RP-HPLC gereinigt: Präparative C18-Säule (Vydac, 218TP1022), Flußrate 25 ml/min, 0.1 % Trifluoressigsäure, 0-40 % Acetonitril in 24 min, 40-100 % Acetonitril in 5 min, 100 % Acetonitril in 5 min. Der Produktpeak eluierte bei ca. 20 % Acetonitril. Die Produktfraktionen wurden vereinigt und gefriergetrocknet.

In einer Abwandlung dieser Synthese wird vor der Reinigung mittels RP-HPLC überschüssiges DTDP durch langsame Zugabe von Wasser unter Rühren ausgefällt. Der Niederschlag wird zweimal in Ethanol aufgenommen und erneut durch Zugabe von Wasser ausgefällt. Die vereinigten wässrigen Phasen werden wie oben mittels RP-HPLC gereinigt.

b) Synthese von 3-(2'-thio-pyridyl)-mercaptopropionyl-glutaminsäure (2b):

Das in a) erhaltene Produkt 1 (s.Fig.1; 0.5 mmol) wurde in 25 ml Dichlormethan gelöst. Unter Rühren und Eiskühlung wurden in einem 50 ml Polypropylenröhrchen (Fa. Peske) der Reihe nach je ein mmol Glutaminsäure-di-t-butylester (Glu(OtBu)OtBu, Bachem), 1-Hydroxybenzotriazol (Aldrich), N-Ethyl-N'-(dimethylaminopropyl)-carbodiimid (Aldrich) und Diisopropylethylamin (Aldrich) zugesetzt. Nach 48 h Reaktion wurde das Gemisch am Rotationsverdampfer bis auf einen öligen Rückstand eingeengt, der in 20 ml Ethylacetat aufgenommen wurde. Diese Lösung wurde je zweimal mit 0.5 M Salzsäure, gesättigter Natriumhydrogencarbonatlösung und gesättigter Natriumchloridlösung ausgeschüttelt. Die organische Phase wurde am Rotationsverdampfer bis auf einen öligen Rückstand eingeengt und über Nacht am Hochvakuum getrocknet (Produkt 2a; s. Fig. 1). Produkt 2a wurde ohne weitere Aufreinigung zur Entfernung der t-Butylschutzgruppen in 30 ml Dichlormethan:Trifluoressigsäure (2:1) aufgenommen und zwei Stunden bei Raumtemperatur gerührt. Am Rotationsverdampfer wurde bis auf einen öligen Rückstand eingeengt, der mit eiskaltem Ether gewaschen wurde. Nach Trocknung am Hochvakuum wurde das Produkt in 100 mM Hepes pH 7.4 gelöst und in Aliquots mittels RP-HPLC (gleiche Bedingungen wie für Produkt 1) gereinigt. Die Produktfraktionen wurden vereinigt. Produkt 2b (s. Fig.1) wurde in einer Ausbeute von 270 µmol erhalten (27 % über alle Stufen).

Theoretisches Molekulargewicht: 344.05.
Gefunden: 345,0 ($MH^+$).

cl) Copolymerisation von Pyridyl-(2-dithiopropionyl)-glutaminsäure (2b) mit O,O'-Bis(2-aminoethyl)poly(ethylenglykol) 6000 (Diamino-PEG-6000; Fluka)

Produkt 2b wurde in 3 ml Dimethylformamid (Fluka) gelöst und mit Dichlormethan auf 20 ml aufgefüllt. 5 ml dieser Lösung (67.5 μmol) wurden der Reihe nach mit 506 mg Diamino-PEG-6000 (84 μmol, entspricht 1.25 Äquivalenten; Fluka), 30 mg Dicyclohexylcarbodiimid (135 μmol, 2 Äquivalente, 135 μl einer 1 M Lösung in DMF) und 2 mg Dimethylaminopyridin (0.25 Äquivalente, 1 M Lösung in DMF) versetzt. Nach 2 h wurden 10 μl für einen Ninhydrintest entfernt, der nur noch schwache Blaufärbung zeigte. Rohprodukt 3 (s. Fig. 1) wurde nach Kühlung auf -20°C durch Fällung aus dem Reaktionsgemisch mit t-Butyl-methylether unter Rühren erhalten. Das Produkt wurde im Vakuum getrocknet. Aliquots wurden in Wasser aufgenommen, und nach Entfernung von unlöslichem Rückstand durch Filtration (Ultra-Free MC, Millipore) wurde mittels Gelfiltration gereinigt. Dazu wurde eine XK 16/40-Säule (Pharmacia) mit Superdex 75 (Pharmacia) nach den Vorgaben des Herstellers gefüllt. Aliquots von je 20 mg Rohprodukt 3 wurden bei einer Flußrate von 1 ml/min mit 20 mM Hepes pH 7.3 als Eluens gereinigt. Die Hauptfraktion eluierte bei einem scheinbaren Molekulargewicht von 40.000 Da nach höhermolekularen, deutlich abgetrennten Vorfraktionen, die getrennt gesammelt wurden.

c2) Copolymerisation von Pyridyl-(2-dithiopropionyl)-glutaminsäure (2b) mit O,O'-Bis(2-aminoethyl)poly(ethylenglykol) 3400 (Diamino-PEG-3400; Fluka) (Produkt 4; s. Fig.1)

Produkt 4 wurde nach gleichem Ansatz und gleicher Aufreinigung erhalten wie Produkt 3. Als Hauptfraktion (54 % der Produktfraktionen) nach Gelfiltration wurde ein Produkt erhalten, das bei einem scheinbaren Molekulargewicht von 22.800 Da eluierte (Nebenfraktionen sind ein Produkt mit 64 kD, 14 % der Gesamtmenge, und ein Produkt mit 46 kD, 32 % der Gesamtmenge).

Das Reaktionsschema der Syntheseschritte a) bis c), die das Copolymergerüst ergeben, ist in Fig. 1 dargestellt: 3-Mercaptopropionsäure wird mit 2,2'-Dithiodipyridin umgesetzt, das Produkt (1) wird an carboxylgeschützte Glutaminsäure gekoppelt (Produkt 2a). Nach Abspaltung der t-Butyl-Schutzgruppen erhält man 3-(2'-thio-pyridyl)-mercaptopropionyl-glutaminsäure (2b), die unter DCC-Aktivierung mit O,O'-Bis(2-aminoethyl)poly(ethylenglykol) 6000 oder mit O,O'-Bis(2-aminoethyl)poly(ethylenglykol) 3400 copolymerisiert wird. Erhalten werden die Produkte 3 bzw. 4.

1.2. Peptidsynthese

Die Peptide wurden nach dem FastMoc™-Protokoll auf einem Applied Biosystems 431A Peptidsynthesizer hergestellt.

i) Peptid YE5C (Sequenz $[\text{Ac-YEEEEE}]_2$-ahx-C) wurde unter Verwendung von 330 mg cysteinbeladenem Chlortritylharz (0.5 mmol/g; Bachem) mit den Schutzgruppen Trityl- (Cys), di-Fmoc (Lys) und O-t-Butyl- (Glu) hergestellt. Es wurde je 1 mmol der geschützten Aminosäuren eingesetzt. Nach dem Verzweigungspunkt (Lys) wurden durchgehend Doppelkopplungen durchgeführt. Die Acetylierung der N-Termini wurde am Peptidharz mit 2 mmol Acetanhydrid in 2 ml N-Methylpyrrolidon in Gegenwart von 2 mmol Diisopropylethylamin durchgeführt. Das Peptid wurde als Rohprodukt nach Spaltung vom Harz (500 μl Wasser, 500 μl Thioanisol, 250 μl Ethandithiol in 10 ml Trifluoressigsäure) und Fällung mit Diethylether gewonnen. Das Rohprodukt wurde in 100 mM HEPES pH 7.9 gelöst und mittels Perfusionschromatographie gereinigt (Poros 20 HQ, Boehringer Mannheim, gefüllt in eine 4 x 100 mm PEEK-Säule. 0 - 0.5 M NaCl in 8 min, Flußrate 10 ml/min). Der Extinktionskoeffizient des Peptids in 50 mM Natriumphosphatpuffer in 6 M Guanidiniumhydrochlorid beträgt bei 280 nm 2560 $M^{-1}cm^{-1}$ (Gill und von Hippel 1989).

ii) Peptid INF7 (Sequenz GLFEAIEGFIENGWEGMIDGWYGC) wurde nach der gleichen Prozedur an 500 mg Chlortritylharz (0.5 mmol/g) synthetisiert, wie für YE5C beschrieben vom Harz gespalten und mit Diethylether gefällt. Das Rohprodukt wurde im Vakuum getrocknet. Aliquots von je 20 mg wurden in 500 μl 1 M Triethylammoniumhydrogencarbonat-Puffer pH 8 gelöst und durch Gelfiltration gereinigt (Sephadex G-10 von Pharmacia gefüllt in eine HR 10/30-Säule von Pharmacia. Flußrate 1 ml/min. Als Eluent dienten 20 mM HEPES pH 7.3 / 150 mM NaCl oder 100 mM TEAB oder 100 mM Ammoniumhydrogencarbonat). Extinktionskoeffizienten: 278 nm 12600; 279 nm 12665; 280 nm 12660 $M^{-1}cm^{-1}$.

iii) Peptid SFO29-ahx (Sequenz K2K-ahx-C) wurde auf analoge Weise synthetisiert (500 mg Fmoc-Cys(Trt)-Chlortritylharz von Bachem; 0.5 mmol/g) und nach Standardmethoden gereinigt (Sephadex G10 mit 0.1 % TFA als

Eluens; Reverse Phase HPLC, 0.1 % TFA - Acetonitril-Gradient). Das Lysin am Verzweigungspunkt war alpha, epsilon-di-Fmoc-L-Lysin, die folgenden Lysine alpha-Fmoc-epsilon-Boc-L-Lysin.

iv) Peptid E4E (Sequenz $[EEEE]_2KGGE$) wurde analog synthetisiert. Ansatzgröße 0.25 mmol Fmoc-Glu(OBzl)-Chlortritylharz. Die Belegung des Harzes erfolgte durch Suspendieren entsprechender Mengen O-Chlorotritylchloridharz (Alexis) in absolutiertem Dichlormethan und Versetzten mit je 2 eq. FmocGlu(OBzl)OH und Diisopropylethylamin. Nach mehrstündigem Schütteln wurde abfiltriert und mehrfach mit Dimethylformamid, Methanol, Isopropanol, Dichlormethan und Diethylether gewaschen. Zum Einsatz kommt ein modifiziertes Fmoc-Protokoll. Die N-terminale Aminosäure trägt eine Boc-Schutzgruppe, um aus der Festphasensynthese ein vollgeschütztes basenstabiles Peptidderivat der Sequenz $(E(Boc)[E(tBu)]_3)_2KGGE(OBzl)OH$ ($E4E^{PROT}$) zu erhalten.

Die Abspaltung vom Harz erfolgte mit Dichlormethan / Essigsäure / Trifluorethanol 8:1:1 bei Raumtemperatur. Die Benzylester-Schutzgruppe der C-terminalen Glutaminsäure wurde selektiv mit $H_2$ / Palladium auf Aktivkohle nach Standardvorschrift abgespalten.

Die Peptidmassen wurden mittels Elektrospray Massenspektroskopie bestimmt und damit die Identität der Peptide bestätigt.

1.3. Ankoppelung der Peptide an das Copolymergerüst (4) bzw. (5)

Die Lösungen von je 1,2 Äquivalenten C-terminal cysteinhaltigem Peptid und Copolymergerüst, erhalten in 1.1 (bezogen auf die Thiopyridyl-Gruppen am Polymer), in 20 mM Hepes, pH 7.4 werden vereinigt und 15 Stunden bei Raumtemperatur gerührt oder geschüttelt.

Zur Bestimmung der einzusetzenden Äquivalente werden die verfügbaren Thiopyridyl-Bindungsstellen durch Versetzen einer verdünnten Lösung des Polymers mit 2-Mercaptoethanol und anschließender Messung der Extinktion des freigesetzten 2-Thiopyridons bei einer Wellenlänge von 342 nm, die Konzentration der freien Thiolfunktionen des cysteinhaltigen Peptides mit Ellman-Reagenz bei einer Wellenlänge von 412 nm nach Lambert-Beer bestimmt.

Nach Ablauf der Reaktion, deren Vollständigkeit durch die Absorption des freigesetzten Thiopyridons bei 342 nm bestimmt wurde, wurde eingeengt und das Produkt mittels Gelfiltration (Superdex 75-Material, Pharmacia) fraktioniert.

1.3.1 Herstellung des Copolymer P3YE5C

Es wurde das verzweigte Peptid YE5C, Sequenz $(YEEEEE)_2K(ahx)C$, verwendet, das über eine Disulfidbrükke des Cystein-Thiols mit der 3-Mercaptopropionyl-glutaminsäure-Gruppierung verknüpft ist.

a) Copolymer P3YE5C wurde aus Fraktion 3 (22.800 Da) des Produktes (4) und gereinigtem Peptid hergestellt. Als Produkt wurde eine Verbindung mit einem scheinbaren Molekulargewicht von 35.000 Da erhalten. Unter Berücksichtigung des Molekulargewichts des Peptides und des Ausgangspolymers bedeutet dies einen Polymerisationsgrad p = 6 (6 sich wiederholende Einheiten).

b) Copolymer P6YE5C wurde aus Fraktion 3 (40.200 Da) des Produktes (3) und gereinigtem Peptid hergestellt. Als Produkt wurde eine Verbindung mit scheinbarem Molekulargewicht 55.800 Da erhalten. Der Polymerisationsgrad ist ca. 7.

1.3.2 Herstellung des Copolymer P3INF7

Es wurde das endosomolytische Peptid INF7 verwendet, das über eine Disulfidbrücke des Cystein-Thiols mit der 3-Mercapto-propionyl-glutaminsäure-Gruppierung verknüpft ist.

a) Copolymer P3INF7 wurde aus Fraktion 3 (22.800 Da) des Produktes (4) und gereinigtem Influenzapeptid hergestellt.

b) Copolymer P6INF7 wurde aus Fraktion 3 (40.200 Da) des Produktes (3) und gereinigtem Influenzapeptid INF7 hergestellt.

1.3.3 Herstellung eines Rezeptorligand-modifizierten ("lactosylierten") Copolymer

Es wurden ein Teil des lactosylierten Pepitds SFO29-ahx und 9 Teile des verzweigten Peptids YE5C, die über

eine Disulfidbrücke des Cystein-Thiols mit der 3-Mercaptopropionyl-glutaminsäure-Gruppierung verknüpft sind, verwendet.

Jeweils 3.32 µmol Copolymer (4) bzw (5) (bezogen auf die enthaltenen Thiopyridyl-Gruppen) gelöst in 1 ml 20 mM HEPES pH 7.4 wurden mit einer Mischung von 500 nmol lactosyliertem SFO29-ahx und 4,48 µmol Peptid YE5C in 1.1 ml HEPES-Puffer versetzt. Dies entspricht einem 1.5-fachen Überschuß an freien Thiolgruppen der Peptide über die zu Verfügung stehenden Thiopyridylgruppen, und der Anteil des lactosylierten Peptids an Gesamtpeptid ist 10 %. Die Reaktion verlief über Nacht vollständig. Die Produkte wurden wie beschrieben mittels Gelfiltration (Superdex 75) gereinigt.

Das Reaktionsschema zur Ankoppelung der geladenen Peptide an das Copolymergerüst gemäß 1.3 ist in Fig. 2 dargestellt: An Produkt 3 bzw. 4 werden Peptide mit freien Thiolgruppen gekoppelt, z.B. das Peptid INF7 (links) oder das Peptid YE5C. Es werden die Produkte P3INF7 (aus O,O'-Bis(2-aminoethyl)poly(ethylenglykol) 3400), P6INF7 (aus O,O'-Bis(2-aminoethyl)poly(ethylenglykol) 6000) und analog P3YE5C und P6YE5C erhalten.

Beispiel 2

Herstellung des Copoylmergerüsts aus Fmoc-6-Aminohexanoyl-glutaminsäure und O,O'-Bis(2-aminoethyl) poly(ethylenglykol) 6000 (Diamino-PEG-6000; Fluka) oder O,O'-Bis(2-aminoethyl)poly(ethylenglykol) 3400 (Diamino-PEG-3400; Fluka).

In diesem Fall gilt für die allgemeine Formel I:

W=Y=NH; X=Fmoc-6-Aminohexanoyl-glutaminsäure.

Das heißt, X ist gemäß i) ein Aminosäurederivat, das durch Ankoppelung von Fmoc-6-Aminohexansäure an Glutaminsäure erhalten wurde. Für die Ankoppelung des Effektormoleküls E kann Z entfallen oder ein bifunktioneller Linker wie SPDP oder EMCS sein.

Ein zur Ankoppelung an dieses Polymergerüst geeigneter Effektor E kann ein Peptid des Typs $E4E^{PROT}$ (Z entfällt) oder des Typs YE5C sein, wobei dieses über das Cystein-Thiol mit dem Linkermolekül Z (z.B. SPDP oder EMCS) reagiert.

a) Synthese des Dipeptids Fmoc-6-Aminohexansäure-GluOH (6)

1 g Fmoc-geschützte 6-Aminohexansäure (2.82 mmol), 1.2 eq. Glu(OtBu)OtBu und 1.2 eq. 1-Hydroxybenzotriazol werden in 200 ml Dichlormethan gelöst. Die Mischung wurde nach Kühlen auf 0°C mit 1.2 eq. N-Ethyl-N'-(dimethylaminopropyl)-carbodiimid und 1.7 ml Diisopropylethylamin versetzt (pH = 8). Nach einer Stunde bei 0°C wurde noch 18 Std bei Raumtemperatur gerührt. Das Lösungsmittel wurde nun vollständig abdestilliert, der Rückstand mit Ethylacetat aufgenommen und mit 0.5 N Salzsäure, gesättigter Natriumhydrogencarbonat-Lösung und gesättigter Natriumchlorid-Lösung extrahiert. Nach Abziehen des Lösungsmittels wurde Fmoc-6-Aminohexansäure-Glu(OtBu)OtBu (5) nach Gefriertocknung erhalten.

Di-t-butyl-geschützes Derivat (5) wurde in 30 ml Dichlormethan/Trifluoressigsäure 2:1 gelöst und eine Stunde bei Raumtemperatur gerührt. Nach vollständiger Reaktion (Reaktionskontrolle mit reversed phase-HPLC) wurde das Lösungsmittel auf ca. 5% des Ausgangsvolumen reduziert und das Produkt (6) durch Präzipitation aus Diethylether erhalten. Abschließende Reinigung erfolgte durch RP-HPLC mit einem Acetonitril/ Wasser/ 0.1%TFA Gradienten.

b) Copolymerisation von Fmoc-6-Aminohexansäure-GluOH (6) mit O,O'-Bis(2-aminoethyl)poly(ethylenglykol) 3400' (Diamino-PEG-3400, Fluka), Produkt (7)

10 mg (6), 1.5 eq. O,O'-Bis(2-aminoethyl)poly(ethylenglykol) 3400', 2 eq. Dicyclohexylcarbodiimid und 0.25 eq. 4-(Dimethylamino)-pyridin werden in 5 ml Dichlormethan gelöst. Nach 30 minütigem Rühren bei Raumtemperatur wurde die Lösung nach Aufkonzentrieren filtriert und das Lösungsmittel danach vollständig abdestilliert. Der Rückstand wurde in 500 µl Wasser suspendiert und gefriergetrocknet.

Nach Abspaltung der Fmoc-Schutzgruppe (20% Piperidin in Dimethylformamid oder Dichlormethan) vom Polymer kann das Copolymer unter Verwendung gängiger Peptid-Kupplungschemie mit beliebigen Peptiden mit freiem C-Terminus conjugiert werden.

Beispiel 3

Herstellung des Copolymergerüsts aus dem geschützten Peptid $E4E^{PROT}$ und O,O'-Bis(2-aminoethyl)poly (ethylenglykol) 6000 (Diamino-PEG-6000; Fluka oder O,O'-Bis(2-aminoethyl)poly(ethylenglykol) 3400 (Diamino-PEG-3400; Fluka)

In diesem Fall ist in der allgemeinen Formel I

W=Y=NH; X = das verzweigte Peptid $E4E^{PROT}$.

In diesem Beispiel stellt ein polyanionisches Peptid X gemäß i) selbst den Effektor dar; daher entfallen Z und E (m = n = 0)

Coolymerisation von $E4E^{PROT}$ mit O,O'-Bis (2-aminoethyl)-poly-(ethylenglykol) 6000' (Diamino-PEG-6000, Fluka) (8);
50 µmol $E4E^{PROT}$, 1.5 eq. O,O'-Bis(2-aminoethyl)-poly(ethylenglykol) 6000', 2 eq. Dicyclohexylcarbodiimid und 0.25 eq. 4-(Dimethylamino)pyridin wurden in 10 ml Dichlormethan gelöst. Nach vierstündigem Rühren bei 4°C wurde die Lösung nach Aufkonzentrieren filtriert und das Lösungsmittel vollständig abdestilliert. Der Rückstand wurde in 500 µl Wasser suspendiert und gefriergetrocknet.

Zur Abspaltung der verbliebenen säurelabilen Seitenkettenschutzgruppen wurde wie in der Literatur beschrieben mit Trifluoressigsäure unter Zusatz von bis zu 5% Scavenger (bevorzugt Ethandithiol, Triethylsilan, Thioanisol) versetzt und zwei Stunden bei Raumtemperatur gerührt. Die Isolierung des Rohproduktes erfolgte durch Präzipitation aus Diethylether. Die endgültige Reinigung erfolgte wie oben beschrieben mittels Gelfiltration (Superdex75, Pharmacia).

Fig. 3: zeigt das Reaktionsschema: Die Benzylschutzgruppe an Carboxylat 1 der C-terminalen Glutaminsäure des vollgeschützten Peptids $E4E^{PROT}$ wird selektiv mit $H_2$/Palladium auf Aktivkohle abgespalten. Das Produkt wird unter DCC-Aktivierung mit O,O'-Bis(2-aminoethyl)poly(ethylenglykol) 6000 oder mit O,O'-Bis(2-aminoethyl)poly(ethylenglykol) 3400 copolymerisiert. Im letzten Schritt werden die Schutzgruppen der N-terminal gelegenen Glutaminsäuren mit TFA in DCM abgespalten.

Beispiel 4

Komplementaktivierungsstudien

Der Test wurde im wesentlichen wie in Plank et al., 1996, beschrieben durchgeführt.

a) Polylysin-DNA-Komplexe mit und ohne Copolymer P6INF7

Polylysin (durchschn. Kettenlänge 170; Sigma) - DNA wurde als Stock-Lösung hergestellt, indem 64 µg pCMVLuc in 800 µl HBS zu 256 µg pL in 800 µl HBS gegeben wurden und durch Pipettieren gemixt wurden, dies entspricht einem berechneten Ladungsverhältnis von 6.3. Von dieser Suspension von Polyplexen wurden als Positivkontrolle je 50 µl in Spalte 1 A-F einer 96-Kalottenplatte gegeben und mit 100 µl $GVB^{2+}$-Puffer versetzt. Alle anderen Vertiefungen enthielten 50 µl $GVB^{2+}$-Puffer. 100 µl wurden von Spalte 1 in Spalte 2 transferiert, gemixt usw. wie in Plank et al. 1996 beschrieben.

Desweiteren wurden je 350 µl der Polylysin-DNA Stock-Lösung versetzt mit 35, 70 und 105 nmol (bezogen auf Anteil INF7) des Polymers P6INF7 und nach 15 min Inkubation mit $GVB^{2+}$-Puffer auf 1050 µl aufgefüllt. Je 150 µl der resultierenden Suspension wurden auf die Reihen A bis F der Spalte 1 einer 96-Kalottenplatte aufgefüllt. Eine 1.5-fache Verdünnungsreihe in $GVB^{2+}$-Puffer und der weitere Komplementaktivierungstest wurden wie oben und in Plank et al. 1996 ausgeführt. Die Endkonzentrationen der Komponenten in Spalte 1 sind für DNA 2/3 µg, für pL 8/3 µg und für das Polymer 0, 5, 10, 15 nmol (bezogen auf INF7) pro 200 µl Gesamtvolumen.

b) Komplementaktivierung durch PEI-DNA-Komplexe mit und ohne Copolymerhülle

Der Test wurde wie beschrieben durchgeführt.

PEI (25 kD, Aldrich) - DNA-Komplexe wurden durch Vereinigung gleicher Volumina einer DNA-Lösung (80 µg/ml in 20 mM HEPES pH 7.4) und einer PEI-Lösung (83,4 µg/ml in 20 mM HEPES pH 7.4) hergestellt. Die DNA-Komplexe wurden zur Entfernung von überschüssigem, ungebundenen PEI drei mal 15 min bei 350 x g in Centricon-100-Filterröhrchen (Millipore) zentrifugiert, wobei zwischen Zentrifugationen jeweils mit in 20 mM HEPES pH 7.4 auf das ursprüngliche Volumen aufgefüllt wurde. Nach dem letzten Zentrifugationsschritt wurde eine Stocklösung an DNA-Komplex erhalten, die einer DNA-Konzentration von 300 µg/ml entsprach. 182 µl dieser Lösung wurden mit 20 mM HEPES pH 7.4 auf 2520 µl verdünnt. Je 610 µl dieser Lösung (entsprechend je 13,2 µg DNA) wurden zu Lösungen von P6YE5C in je 277,6 µl 20 mM HEPES pH 7.4 pipettiert. Die resultierenden Lösungen wurden mit 5 M NaCl auf eine Salzkonzentration von 150 mM gebracht. Je 150 µl der resultierenden Lösungen wurden in Spalte 1, A bis F, einer 96-Well-Platte transferiert. Die Verdünnungsreihe in $GVB^{2+}$-Puffer wurde wie beschrieben durchgeführt (Plank et al. 1996).

Ebenso wurden je 610 pl PEI-DNA-Komplex höherer Konzentration (86 ng DNA pro µl) mit je 277,6 µl von Lösungen des Polymers P3YE5C inkubiert. Die Lösungen enthielten 0, 1, 2 ,3 Ladungsäquivalente bezüglich der eingesetzten DNA an Peptid YE5C. Nach 15 min wurden je 27,45 µl 5 M NaCl zugefügt (resultierendes Gesamtvolumen 915 µl). Je 150 µl der resultierenden Lösung wurden in Spalte 1, Reihen A bis F einer 96-Kalottenplatte transferiert

(dies entspricht jeweils 8.6 µg DNA und 9 µg PEI). Die Verdünnungsreihe in $GVB^{2+}$ und der weitere Versuchsvorgang wurde wie oben für pL-DNA beschrieben durchgeführt.

Das Ergebnis des Komplementaktivierungstests ist in Fig. 4 dargestellt:

A) Komplementaktivierung durch Polylysin-DNA-Komplexe in Anwesenheit und Abwesenheit des Copolymers P6INF7. Der CH50-Wert bezeichnet eine bestimmte Serumverdünnung, die zur Lyse von 50 % der Schaferythrozyten in diesem Versuchsaufbau führt. Der Wert $CH50_{max}$ bezeichnet jenen CH50-Wert, der mit unbehandeltem Humanserum erhalten wird. Im beschriebenen Versuchsaufbau wurde Humanserum mit Genvektoren inkubiert. Die CH50-Werte, die mit so behandeltem Serum erhalten werden, sind niedriger als $CH50_{max}$, wenn die Vektoren die Komplementkaskade aktivieren. Die Daten sind in Prozent von $CH50_{max}$ gezeigt. Die starke Komplementaktivierung, die mit Polylysin-DNA-Komplexen beobachtet wurde, kann durch Hüllpolymer P6INF7 gänzlich unterbunden werden.

B) Das Peptid INF7 selbst, in freier Form oder polymergebunden, ist ein schwacher Komplement-Aktivator. Eingebaut in einen Polylysin-DNA-Komplex verschwindet diese Komplementaktivierung.

C) Komplementaktivierung durch PEI-DNA-Komplexe (N/P = 8) in Gegenwart des Copolymers P3YE5C. Der ungeschützte DNA-Komplex ist ein starker Aktivator des Komplementsystems. Das Copolymer P3YE5C vermindert die Komplementaktivierung in Abhängigkeit von der zugesetzten Menge an Copolymer, führt jedoch im untersuchten Bereich nicht zu vollständigem Schutz.

D) Dagegen schützt Copolymer P6YE5C schon in kleinen zugesetzten Mengen vollständig vor Komplementaktivierung.

Beispiel 5

Erythrozyten-Lyse-Test

Der durchgeführte Test dient zur Untersuchung der Fähigkeit von Peptiden, natürliche Membranen pH-abhängig zu lysieren.

Die in diesem Beispiel verwendeten Erythrozyten wurden wie folgt erhalten: 10 ml frisches Blut wurde von Freiwilligen entnommen und sofort in 10 ml Alsevers Lösung (Whaley 1985; Plank et al., 1996) aufgenommen. Aliquots von je 3 ml wurden 3 mal mit dem entsprechenden Puffer (je 40 ml Citrat bzw. HBS) gewaschen (nach Pufferzugabe und Schütteln Zentrifugation bei 2500 x g und Verwerfen des Überstandes). Die Konzentration der Erythrozyten wurde mit einem "Extinktionskoeffizienten" von $2.394 \times 10^{-8}$ ml/Zellen bei 541 nm bestimmt. Zur Herleitung des Extinktionskoeffizienten wurde die Zellzahl in einem Aliquot mit einer Neubauerkammer bestimmt und nachfolgend die Extinktion dieser Lösung nach Zugabe von 1 µl 1 % Triton X-100 bei 541 nm bestimmt.

In einer 96-Kalotten-Platte wurden in Spalte 1 Aliquots von INF7 bzw. copolymergekoppelten INF7 (P6INF7) in 150 µl 10 mM Natriumcitrat pH 5 / 150 mM NaCl bzw. in HBS-Puffer vorgelegt (üblicherweise 45 µmol Peptid). In allen anderen Vertiefungen wurden je 50 µl Puffer (Citrat bzw. HBS) vorgelegt. 100 µl wurden mit einer Mehrkanalpipette von Spalte 1 in Spalte 2 transferiert, durch Pipettieren gemixt, 100 µl wurden von Spalte 2 in Spalte 3 transferiert usw. Die übrigen 100 µl aus Spalte 11 wurden verworfen, in Spalte 12 befand sich nur Puffer. Die resultierende 1.5-fache Verdünnungsreihe wurde mit je 50 µl Puffer (Citrat bzw. HBS) auf 100 µl aufgefüllt. Daraufhin wurden je $3 \times 10^6$ humane Erythrozyten zugegeben, die Platten mit Parafilm abgedeckt und bei 400 rpm in einem Schüttelinkubator (Series 25 Incubator Shaker; New Brunswick Scientific Co.; NJ, USA) bei 37°C für 1 h geschüttelt. Daraufhin wurden die Platten bei 2500 x g zentrifugiert, je 150 µl Überstand in eine Flachboden-96-Kalottenplatte transferiert und freigesetztes Hämoglobin bei 410 nm mit einem ELISA-Plate-Reader gemessen. 100 % Lyse wurde durch Zugabe von 1 µl 1 % Triton X-100 in einzelne Vertiefungen aus Spalte 12 (vor Transferieren in die Flachbodenplatte) bestimmt, 0 % Lyse wurde aus unbehandelten Proben aus Spalte 12 bestimmt.

Das Ergebnis des Erythrozyten-Lyse-Tests ist in Fig. 5 dargestellt: Peptid INF7 zeigte hohe pH-spezifische Aktivität. Aus der Synthese des Copolymers P3INF7 wurden nach chromatographischer Auftrennung (Superdex 75, Pharmacia) vier Fraktionen (abnehmendes Molekulargewicht von 1 bis 4) gewonnen. Von diesen zeigten Fraktionen 2 und 3 gegenüber freiem Peptid INF7 höhere Lyseaktivität. In allen Fällen war die Aktivität streng pH-abhängig, d.h. keine Lyse bei neutralem pH (nicht gezeigt).

Beispiel 6

Größenbestimmung von DNA-Komplexen mittels dynamischer Lichtstreuung und Elektronenmikroskopie

Herstellung von PEI-DNA Polyplexen und Aufbringen der Polymerhülle: Je 40 µg DNA (pCMVLuc) in 333 µl 20 mM HEPES pH 7.4 wurden zu 41.7 µg PEI (25 kD, Aldrich) in 333 µl HEPES pH 7.4 pipettiert und gemixt. Nach 10 - 15 min Inkubation wurden 0, 0.5, 1, 1.5, 2 oder 3 Ladungsäquivalente (bezüglich der Ladung der eingesetzten DNA) an Polymeren P3YE5C bzw. P6YE5C in je 333 µl HEPES zugesetzt (bzw. 0, 1, 2, 3 und 5 Äquivalente in einem zweiten Experiment). Dies entspricht einer Menge bezogen auf das Peptid YE5C von 0, 152, 303, 455, 606 oder 909 pmol an Polymer pro µg DNA. DOTAP/Cholesterin-DNA-Komplexe wurden aus DOTAP/Cholesterin (1:1 mol/mol)-Liposomen in 330 µl 20 mM HEPES pH 7.4 und DNA im selben Volumen bei einem Ladungsverhältnis von 5 hergestellt. Die Lipoplexe wurden mit 0, 1, 2, 3 und 5 Äquivalenten des Copolymers P3YE5C in 330 µl Puffer inkubiert. Die endgültige DNA-Konzentration des Komplexes war 10 µg/ml.

Die Größe der DNA-Komplexe wurde einerseits durch dynamische Lichtstreuung (Zetamaster 3000, Malvern Instruments) sofort nach Polymerzugabe und dann zu verschiedenen Zeitpunkten über mehrere Stunden hinweg bestimmt, andererseits durch Elektronenmikroskopie wie in Erbacher et al., 1998, und Erbacher et al., 1999, beschrieben.

Fig. 6 zeigt die elektronenmikroskopischen Aufnahmen von PEI-DNA-Komplexen (N/P = 8) in Gegenwart des Copolymers P3YE5C.

A) In Gegenwart eines Ladungsäquivalents (bezüglich der Ladungen der eingesetzten DNA) des Copolymers. Die Partikelgröße beträgt 20 bis 30 nm.

B) In Gegenwart von zwei Ladungsäquivalenten des Copolymers. Die Mehrzahl der Partikel weist Größen um 20 nm auf. Dies sind monomolekulare DNA-Komplexe, d.h. ein Plasmidmolekül verpackt in ein Partikel.

C) In Gegenwart von 1,5 Ladungsäquivalenten des Copolymers nach Zugabe von BSA zu einer Endkonzentration von 1 mg/ml und Inkubation über Nacht. Copolymergeschützte DNA-Komplexe bleiben stabil und aggregieren nicht, im Gegensatz zu ungeschützten PEI-DNA-Komplexen, die unter diesen Bedingungen sofort präzipitieren (nicht gezeigt).

Beispiel 7

Bestimmung des Zetapotentials von DNA-Komplexen

An den gleichen Proben wie in Beispiel 6 wurden die Zetapotentiale mit dem Gerät von Malvern bestimmt, wobei die Parameter Brechungsindex, Viskosität und Dielektrizitätskonstante auf die Werte von deionisiertem Wasser eingestellt wurden, was nur näherungsweise gelten kann.

Fig. 7 zeigt das Zetapotential von PEI- und DOTAP/Cholesterin-DNA-Komplexen in Abhängigkeit von der Menge zugesetzten Copolymers P3YE5C. Das Zetapotential als Maß für die Oberflächenladung der Komplexe nimmt von stark positiv über neutral zu leicht negativ mit steigender Menge zugesetzten Copolymers ab. Dies zeigt, daß das Copolymer an die DNA-Komplexe bindet und deren elektrostatische Ladung ausgleicht bzw. abschirmt.

Beispiel 8

Herstellung von DNA-Komplexen und Transfektionen

Für die in den nachfolgenden Beispielen durchgeführten Zellkultur- und Transfektionsexperimente wurden, wenn nich anders angegeben, die folgenden Materialien und Methoden verwendet:

a) Gentransfer in Zellkultur in einer 96-Well Platte

Adhärente Zellen werden am Tag vor der Transfektion in Flachboden-Platten zu 20.000 - 30.000 Zellen pro Vertiefung ausgesät (je nach Teilungsrate der Zellen. Während der Transfektion sollte die Konfluenz etwa 70 - 80 % sein).

Vor der Transfektion wird Medium abgesaugt. Zur Transfektion gibt man 150 µl Medium auf die Zellen und gibt dann 50 µl DNA-Komplexe zu.

b) Zusammensetzung der DNA-Komplexe: Vorzugsweise 1 µg DNA/well Endkonzentration; Berechnung für 1.2-fache Menge; 20 µl Volumen pro Komponente (DNA, PEI, Polymer). Schließlich werden 50 µl DNA-Komplex für die

Transfektion verwendet. Puffer: 20 mM HEPES pH 7.4/ 150 mM NaCl = HBS. Die verwendeten Volumina an Puffer bleiben gleich.

Für den Fall, daß man DNA-Komplexe für 96 Einzelversuche benötigt, führt man die Berechung zweckmäßig so durch, als würde man 100 Einzelversuche durchführen: z.B.

DNA: 1 µg x 100 x 1.2 = 120 µg in 20 x 100 µl HBS = 2 ml Gesamtvolumen.

Polyethylenimin (PEI): Um ein N/P-Verhältnis von 8 zu erzielen berechnet man gemäß der Formel

$$N/P = \frac{(\mu g PEI)}{43} \times \frac{330}{(\mu g DNA)}$$

$$8 = \frac{(\mu g PEI)}{43} \times \frac{330}{(120)},$$

daß man 125.09 µg PEI benötigt und zwar in 20 x 100 µl = 2 ml HBS.

Hüllpolymer: Will man z.B. für die angegebene Menge DNA und PEI Hüllpolymer in einer Menge von 2 Ladungsäquivaltenten (bezüglich DNA) einsetzen so berechnet sich nach der Formel

$$\mu l(\text{Hüllpol.}) = 1000 \times \frac{(\mu g DNA)}{330} \times \frac{\textit{Ladungsäqu.}}{c\ (\text{Hüllpol. } [\mu mol/ml])}$$

das benötigte Volumen an Hüllpolymer bei einer Konzentration c von 11.1 µmol / ml als

$$\mu l(\text{Hüllpol.}) = 1000 \times \frac{120}{330} \times \frac{2}{11.1} = 65.5\ \mu l,$$

die ebenfalls auf 2 ml mit HBS aufgefüllt werden.

Dies ist ein Beispiel für 100 Versuche zu je 1 µg DNA. Üblicherweise werden etwa je 5 Versuche durchgeführt und z.B. N/P-Verhältnisse von 4, 5, 6, 7, 8 mit jeweils 0, 1, 2, 3 Ladungsäquivalenten Hüllpolymer untersucht.

c) Mischen der DNA-Komplexe:

Nach Herstellung der benötigten Vorverdünnungen wird DNA unter Vortexen zu PEI gegeben. Nach 15 min wird wiederum unter Vortexen Hüllpolymer zum vorgefertigten PEI-DNA-Komplex gegeben. Nach weiteren 30 min werden je 50 µl DNA-Komplexe zu den Zellen gegeben, die sich in je 150 µl Medium befinden.

Die verwendeten Gefäße richten sich nach dem berechneten Gesamtvolumen. Im obigen Fall wird PEI zweckmäßig in einem 14 ml Polypropylen-Röhrchen (z.B. Falcon 2059) vorgelegt, die anderen beiden Komponenten in 6 ml-Röhrchen (z.B. Falcon 2063). Für Einzelversuche im 96-Well-Plate kann man die Komponenten auch in einer 96-Well-Platte anmischen. Wenn das endgültige

Gesamtvolumen der DNA-Komplexe 1 - 1.5 ml beträgt, eignen sich Eppendorf-Tubes. Zum Mischen kann statt Vortexen mit der Mikropipette auf- und abpipettiert werden.

Umrechnung auf 3 cm-Schalen (6-well plate):

Für 3 cm Schalen (6-well plate) werden zweckmäßig Mengen von 2 bis 5 µg DNA, Volumen je Komponente etwa je 100 µl eingesetzt, wobei die Berechnung analog zu oben erfolgt. Im 12-well plate werden zweckmäßig Mengen von ca. 1 µg DNA je Versuch eingesetzt.

Fig. 8 zeigt schematisch die Formulierung von DNA-Komplexen: Bevorzugt wird zuerst Polykation mit Plasmid-DNA inkubiert, was zu einem positiv geladenen DNA-Komplex führt (z.B. PEI, N/P = 8). Dann wird negativ geladenes Copolymer zugesetzt, das elektrostatisch an den vorgeformten Komplex bindet. Das Copolymer kann mit Rezeptorliganden modifiziert sein, symbolisiert durch Sterne (rechts).

d) Luciferin-Substratpuffer

Als Luciferin-Substratpuffer wurde eine Mischung von 60 mM Dithiothreitol, 10 mM Magnesiumsulfat, 1 mM ATP, 30 µM D (-)-Luciferin, in 25 mM Glycyl-Glycin-Puffer pH 7.8, verwendet.

e) Proteinbestimmung in Zellysaten

Den Proteingehalt der Lysate bestimmte man mit dem Bio-Rad Protein Assay (Fa. Bio-Ra): Zu 10 µl (bzw. 5 µl) des Lysats wurden 150 µl (bzw. 155 µl) dest. Wasser und 40 µl Bio-Rad Protein Assay Farbstoff-Konzentrat in eine Vertiefung einer durchsichtigen 96-Kalotten-Platte (Typ "flat bottom", Fa. Nunc, Dänemark) gegeben und bei 630 nm mit dem Absorbance-Reader "Biolumin 690" und dem Computer-Programm "Xperiment" (beide Fa. Molecular Dynamics, USA) die Absorption gemessen. Als Eichkurve wurden Konzentrationen von 50, 33.3, 22.3, 15,

9.9, 6.6, 4.4, 2.9, 2.0, 1.3, 0.9 und 0 ng BSA / µl vermessen. Bovines Serum Albumin (BSA) wurde als Bio-Rad Protein Assay Standard II gekauft. Somit konnte insgesamt eine Angabe in pg Luciferase pro mg Protein gemacht werden.

Beispiel 9

Gentransfer in K562-Zellen mit PEI(25 kD)-DNA-Komplexen in Gegenwart und Abwesenheit des Copolymers P3YE5C K562-Zellen (ATCC CCL 243) wurden in RPMI-1640-Medium unter Zusatz von 10 % FCS, 100 units/ml Penicillin, 100 µg/ml Streptomycin und 2 mM Glutamin bei 37°C in einer Atmosphäre von 5 % $CO_2$ kultiviert. Die Zellen wurden am Abend vor der Transfektion mit Desferoxamin zu einer Endkonzentration von 10 µM versetzt. Unmittelbar vor der Transfektion wurde das Medium gewechselt. Je 50.000 Zellen in je 160 µl Medium wurden in die Vertiefungen einer 96-Well-Platte ausgebracht.

Transferrin-PEI (hTf-PEI 25 kD) wurde im wesentlichen wie von Kircheis et al. beschrieben (Kircheis et al., 1997) durch reduktive Aminierung hergestellt. Es wurde ein Produkt erhalten, das durchschnittlich 1.7 Transferrinmoleküle pro PEI-Molekül gekoppelt hat. In einem Vorversuch wurde eine Zusammensetzung von hTf-PEI-Polyplexen bestimmt, die hohe Transfektion ergibt und bei der ein deutlicher Einfluß des Rezeptorliganden feststellbar ist. 32.4 µg hTf-PEI (Mengenangabe bezieht sich auf hTf) in 600 µl HBS wurden mit 36 µg PEI (25kD) in 600 µl HBS vereinigt. Zu dieser Mischung wurden 40 µg DNA (pCMVLuc) in 600 µl HBS pipettiert und gemischt. Je 270 µl der resultierenden Lösung wurden nach 15 min zu je 90 µl von Lösungen des Polymers P3YE5C in HBS bzw. HBS alleine gegeben. Diese Lösungen enthielten Polymermengen, die 0/0.5/1/1.5/2/3 Ladungsäquivalenten bezüglich der Ladung der eingesetzten DNA enthielten. Gleichermaßen wurden DNA-Komplexe ohne hTf mit der äquivalenten Menge PEI hergestellt (40 µg DNA + 42 µg PEI + Hüllpolymer). Je 60 µl der resultierenden Mischungen (entspricht einer Menge von je 1 µg DNA / well) wurden in je 5 Vertiefungen einer Rundboden-96-Well-Platte vorgelegt und 50.000 K562 in je 160 µl RPMI-Medium wurden zugegeben. Nach 24 h wurden die Zellen durch Zentrifugation sedimentiert, der Überstand durch Aspiration entfernt und je 100 µl Lysepuffer (250 mM Tris pH 7.8; 0.1 % Triton X-100) wurden zugegeben. Nach 15 min Inkubation wurde durch Pipettieren gemischt und je 10 µl Probe wurden zum Luciferasetest im 96-well-plate-Format in eine schwarze Platte (Costar) transferiert und mit je 100 µl Luciferin-Substrat-Puffer versetzt. Die Messung der resultierenden Lichtemission erfolgte mittels Microplate Scintillation & Luminescence counter "Top Count" (Fa. Canberra-Packard, Dreieich). Die Meßzeit betrug 12 Sekunden, die Meßverzögerung war 10 min. und Hintergrund-Werte wurden automatisch abgezogen. Als Standard wurden je 100, 50, 25, 12.6, 6.25, 3.13, 1.57, 0.78, 0.39, 0.2, 0.1, 0.05, 0.025, 0.013, 0.007 und 0 ng Luciferase (Boehringer Mannheim) in je 10 µl Lysepuffer (= 2-fache Verdünnungsserie) unter gleichen Bedingungen gemessen und daraus eine Eichkurve erstellt.

Fig. 9 zeigt das Ergebnis der Gentransferexperimente mit PEI-DNA-Komplexen (N/P = 8) in K562-Zellen in Gegenwart und Abwesenheit von Transferrin als Rezeptorligand unter Zusatz des Copolymers P3YE5C. Das Copolymer interferiert nicht mit Gentransfer und steigert diesen sogar, wenn ein Rezeptorligand im DNA-Komplex vorhanden ist. Gezeigt ist die Expression des Reportergens Luciferase bezogen auf die Menge Gesamtprotein im Zellextrakt (Mittelwerte und Standardabweichungen aus Triplikaten).

Beispiel 10

Transfektion der Brustkrebszellinie MDA-MB435S mit Polylysin-DNA-Komplexen in Gegenwart und Abwesenheit des Hüllpolymers P3INF7

MDA-MB435S Zellen (ATCC?? humane Brustkrebs-Zellinie) wurden in DMEM-Medium unter Zusatz von 10 % FCS, 100 units/ml Penicillin, 100 µg/ml Streptomycin und 2 mM Glutamine bei 37°C in einer Atmosphäre von 5 % $CO_2$ kultiviert. Am Abend vor der Transfektion wurden die Zellen zu 20.000 Zellen pro Vertiefung in 96-Well-Platten (Flachboden) ausgebracht.

Die DNA-Komplexe wurden wie folgt hergestellt: Berechnung für 1 Well: Zu erzielende Menge ist 1 µg DNA pro Well, 4 µg pL170 in einem Gesamtvolumen von 60 µl HBS. Diese Mengen wurden mit 1.2 multipliziert. Die DNA-Komplexe wurden wie in folgender Tabelle angegeben gemixt, wobei zuerst DNA zu Polylysin gegeben wurde und dies wiederum nach 15 min zu Polymer P3INF7 bzw. zum Puffer. Die Versuche wurden in Triplikaten ausgeführt. Je 60 µl DNA-Komplexe wurden zu Zellen, bedeckt von 150 µl Medium gegeben. Nach 4 h wurde Medium gewechselt, nach 24 h wurde nach Waschen mit PBS und Zugabe von 100 µl Lysepuffer der Luciferasetest und der Proteintest wie in Beispiel 9 beschrieben durchgeführt.

| Nr. | P3INF7 | HBS µl | pL170 µl = µg | HBS | 7.2µg DNA in HBS(µl) |
|---|---|---|---|---|---|
| 1 | 144,6 (5 nmol) | 71,4 | 28,8 | 79,2 | 108 |

(fortgesetzt)

| Nr. | P3INF7 | HBS µl | pL170 µl = µg | HBS | 7.2µg DNA in HBS(µl) |
|---|---|---|---|---|---|
| 2 | 289,2 (10 nmol) | - | 28,8 | 42,6 | 71,4 |
| 3 | - | - | 28,8 | 187, 2 | 216 |

Fig. 10 zeigt das Ergebnis der Gentransferexperimente mit Polylysin-DNA-Komplexen in die humane Brustkrebszellinie MDA-MB435S in Gegenwart und Abwesenheit des Copolymers P3INF7. In Abwesenheit des Copolymers tritt keine meßbare Reportergenexpression auf. Die pH-abhängig membranzerstörende und somit endosomolytische Aktivität des Copolymers bewirkt effizienten Gentransfer. 5 nmol bzw. 10 nmol P3INF7 beziehen sich auf die Menge des eingesetzten, polymergebundenen Peptids INF7.

Beispiel 11

Lipofection in Gegenwart von Hüllpolymeren (Fig. 11)

NIH3T3-Zellen (ATCC CRL 1658) wurden in DMEM-Medium unter Zusatz von 10 % FCS, 100 units/ml Penicillin, 100 µg/ml Streptomycin und 2 mM Glutamine bei 37°C in einer Atmosphäre von 5 % $CO_2$ kultiviert. Am Abend vor der Transfektion in 6-Well-Plates in einer Dichte von 500.000 Zellen pro Vertiefung ausgebracht.

Herstellung von DNA-Komplexen:

16 µg DNA in 240 µl 20 mM HEPES pH 7.4 wurden mit einer Lösung von 242 nmol DOTAP/Cholesterin-Liposomen in 240 µl des gleichen Puffers versetzt. Dies ergibt ein Ladungsverhältnis ($^+/_-$) von 5. 210 µl der resultierenden Lösung wurden zu 105 µl einer Lösung pipettiert, die 6,36 nmol des Polymers P3YE5C enthielt (bezogen auf den Peptidanteil YE5C; dies entspricht 3 DNA-Ladungsäquivalenten). Für den Kontrollversuch wurden 210 µl DOTAP/Cholesterin-DNA zu 105 µl 20 mM HEPES pH 7.4 pipettiert. Je 90 µl der resultierenden DNA-Komplexe wurden den Zellen zugegeben, die sich in 800 µl frischem Medium befanden; dies entspricht 2 µg DNA pro Vertiefung. Die Versuche wurden in Triplikaten ausgeführt.

Gleichermaßen wurde der Versuch mit Lipofectamine™ anstatt DOTAP/Cholesterin durchgeführt. In diesem Falle wurde eine Menge von Lipofectamin (DOSPA) eingesetzt, die zu einem Ladungsverhältnis von 7 ($^+/_-$) führt.

30 min nach Zugabe der DNA-Komplexe wurden den Zellen je 1 ml frisches Medium zugefügt, nach 3 h weitere 2 ml. Das Medium wurde nicht gewechselt. 22 h nach Komplex-Zugabe wurden die Zellen mit PBS gewaschen und in 500 µl Lysepuffer lysiert. Aliquots des Zellysats wurden im Luciferasetest und zur Proteinbestimmung eingesetzt.

Fig. 11 zeigt das Ergebnis der Lipofection in NIH3T3-Zellen in Gegenwart und Abwesenheit des Copolymers P3YE5C. Weder die Transfektion mit DOTAP/Cholesterin-DNA noch jene mit Lipofectamin wird signifikant erniedrigt (3 Ladungsäquivalente des Copolymers; DOTAP/Cholesterin-DNA hat bei dieser Zusammensetzung ein neutrales Zetapotential, s. Fig. 7).

Beispiel 12

Transfektion von HepG2-Zellen mit DOTAP/Cholesterin-DNA und PEI-DNA in Gegenwart und Abwesenheit von P6YE5C

HepG2-Zellen (ATCC HB 8065) wurden in DMEM-Medium unter Zusatz von 10 % FCS, 100 units/ml Penicillin, 100 µg/ml Streptomycin und 2 mM Glutamine bei 37°C in einer Atmosphäre von 5 % $CO_2$ kultiviert. Zwei Tage vor der Transfektion wurden die Zellen in 6-Well-Plates in einer Dichte von 500.000 Zellen pro Vertiefung ausgebracht. Die Transfektion mit DOTAP/Cholesterin erfolgte exakt, wie oben für NIH3T3-Zellen beschrieben, wobei diesmal Polymer P6YE5C verwendet wurde. Weiters wurden 7 µg DNA in 105 µl HEPES-Puffer zu 7,3 µg PEI 25 kD gelöst im gleichen Volumen pipettiert. Nach 15 min Inkubation wurde diese Lösung zu 105 µl einer Lösung des Polymers P6YE5C pipettiert, die 3 DNA-Ladungsäquivalente an YE5C enthielt. Je 90 µl dieser Lösung wurden den Zellen zugegeben. Die Versuche wurden in Triplikaten ausgeführt.

Fig. 12 zeigt den Gentransfer in HepG2-Zellen in Gegenwart und Abwesenheit des Copolymers P6YE5C. Transfektion durch DOTAP/Cholesterin-DNA wird nicht signifikant inhibiert. Die Transfektion durch PEI-DNA-Komplexe wird erniedrigt (3 Ladunsäquivalente des Copolymers).

Beispiel 13

Intravenöser Gentransfer in vivo

a) Kontrolle (PEI-DNA, N/P = 8):
150 µg DNA (pCLuc) in 337.5 µl 20 mM HEPES pH 7.4 wurden zu 156.4 µl PEI (25 kD, Aldrich) im gleichen Volumen HEPES-Puffer pipettiert. Nach 15 min wurden 75 µl 50 % Glucose zugegeben. Von dieser Lösung wurden je 100 µl in die Schwanzvene von Mäusen injiziert (entsprechend einer Dosis von 20 µg DNA pro Tier).

b) Kontrolle (DOTAP/Cholesterin-DNA; Ladungsverhältnis $^{+}/_{-}$ = 5):
DOTAP-Cholesterin-Liposomen wurden nach Standardvorschrift hergestellt (Barron et al., 1998). In diesem Fall wurden Liposomen mit einem molaren Verhältnis DOTAP zu Cholesterin von 1:1 hergestellt und einer Endkonzentration von DOTAP von 5 mM in 5 % Glucose. 130 µg DNA in 91.1 µl 20 mM HEPES pH 7.4 wurden zu 393.5 µl Liposomensuspension gegeben. Nach 15 min wurden 65 µl 50 % Glucose zugegeben. Von dieser Lösung wurden je 100 µl in die Schwanzvene von Mäusen injiziert (entsprechend einer Dosis von 20 µg DNA pro Tier).

c) PEI-DNA (N/P = 8) mit Copolymerhülle:
150 µg DNA in 2475 µl wurden zu 156.4 µg PEI (25 kD) im gleichen Volumen unter Vortexen zugegeben. Nach 15 min wurden 3 Ladungsäquivalente (bezüglich der Ladungen der eingesetzten DNA-Menge) an Polymer P3YE5C in 2475 µl HEPES-Puffer unter Vortexen zugegeben. Nach weiteren 30 min wurden die DNA-Komplexe durch Zentrifugation in Centricon 30-Röhrchen auf eine DNA-Konzentration von 454 µg/ml aufkonzentriert. Diese Lösung wurde dann unter Zugabe von 50 % Glucose und 20 mM HEPES pH 7.4 auf eine Endkonzentration von 200 µg DNA pro ml und 5 % Glucose gebracht. Von dieser Lösung wurden je 100 µl in die Schwanzvene von Mäusen injiziert (entsprechend einer Dosis von 20 µg DNA pro Tier).

d) DOTAP/Cholesterin-DNA (5:1) mit Copolymerhülle:
393.9 µl Liposomensuspension wurden direkt zu einer Lösung von 130 µg DNA in 65.3 µl Wasser pipettiert. Nach 15 min wurden 3 Ladungsäquivalente P3YE5C in 216.9 µl HEPES-Puffer zugegeben und nach weiteren 30 min 75 µl 5 % Glucose. Von dieser Lösung wurden je 115.5 µl in die Schwanzvene von Mäusen injiziert (entsprechend einer Dosis von 20 µg DNA pro Tier).

Fig. 13 zeigt das Ergebnis der in vivo Gentransferexperimente: PEI-DNA- bzw. DOTAP/Cholesterin-DNA-Komplexe mit oder ohne gebundenes Copolymer P3YE5C (3 Ladungsäquivalente) wurden in die Schwanzvene von Mäusen injiziert (n = 6). Die Tiere wurden 24 h nach Injektion geopfert, und die Reportergenexpression in Organen wurde bestimmt. Die höchste Aktivität wurde jeweils an der Injektionsstelle gemessen, mit PEI-DNA-Copolymer trat signifikante Reportergenexpression in Lunge und Herz auf, während Gentransfer in die Lunge durch DOTAP/Cholesterin-DNA bei Anwendung des Copolymers inhibiert wurde.

Beispiel 14

Sterische Stabilisierung von PEI-DNA-Komplexen

PEI-DNA-Komplexe wurden exakt wie in Beispiel 6 beschrieben hergestellt (PEI-DNA, N/P = 8, 0/1,5/3 Ladungsäquivalente Copolymer P3YE5C bzw. P6YE5C). Die Größe der Komplexe wurde mittels dynamischer Lichtstreuung bestimmt und betrug 20 bis 30 nm. Daraufhin wurde 5 M NaCl zu einer Endkonzentration von 150 mM zugegeben. PEI-DNA ohne Copolymer aggregierte sofort (nach 5 min war eine Population von Partikeln >500 nm zu messen; nach 15 min war die Mehrzahl der Partikel >1000 nm; über Nacht fielen die Komplexe aus der Lösung aus). In Gegenwart von P3YE5C bzw. P6YE5C (1,5 oder 3 Ladungsäquivalente) blieb die Partikelgröße zumindest über 3 Tage stabil.

Ebenso führte die Zugabe von BSA zu einer Endkonzentration von 1 mg/ml zu sofortiger Präzipitation von PEI-DNA. In Gegenwart von P3YE5C bzw. P6YE5C (1,5 Ladungsäquivalente und mehr) bleibt die Partikelgröße zumindest über 24 h konstant (siehe auch Fig. 6c).

Literatur

Balkenhohl, F., von dem Bussche-Hünnefeld, C., Lansky, A. and Zechel, C. (1996). Angew. Chem. 108: 2436-2488.

Barron, L. G., Meyer, K. B. and Szoka, F. C. J. (1998). Hum. Gene Ther. 10(9(3)): 315-23.

Boussif, O., Lezoualch, F., Zanta, M. A., Mergny, M. D., Scherman, D., Demeneix, B., & Behr, J. P. (1995) Proc. Natl. Acad. Sci. USA 92, 7297-7301.

Brocchini, S., James, K., Tangpasuthadol, V. and Kohn, J. (1997). J. Am. Chem. Soc. 119(19): 4553-4554.

Coombes, A. G. A., Tasker, S., Lindblad, M., Holmgren, J., Hoste, K., Toncheva, V., Schacht, E., Davies, M. C., Illum, L. and Davis, S. S. (1997). Biomaterials 18(17): 1153-1161.

Erbacher, P., Remy, J.-S. and Behr, J.-P. (1999). Gene Ther. 6(1):138-45

Erbacher, P., Zou, S. M., Bettinger, T., Steffan, A. M. and Remy, J. S. (1998). Pharm. Res. 15(9): 1332-1339.

Felix, A., M., Heimer, E., P., Lambros, T., J., Tzongraki, C., Meienhofer, J. (1978). J. Org. Chem. 43, 4194-4196.

Ferrari, S., Moro, E., Pettenazzo, A., Behr, J. P., Zacchello, F., & Scarpa, M. (1997) Gene Ther. 4, 1100-1106.

Fields, G., B., Noble, R., L. (1990). Int. J. Peptide Protein Res. 35, 161-214.

Fields, G., B., et al., (1991). Pept. Res. 4, 88.

Gill S.C., von Hippel P.H. *Anal. Biochem.* 1989; 182: 319-326

Gottschalk, S., Sparrow, J. T., Hauer, J., Mims, M. P., Leland, F. E., Woo, S. L. C., & Smith, L. C. (1996) Gene Ther. 3, 448-457.

Haensler, J. and Szoka, F. C. (1993). Bioconj. Chem. 4(5): 372-379.

Kircheis, R., Kichler, A., Wallner, G., Kursa, M., Ogris, M., Felzmann, T., Buchberger, M. and Wagner, E. (1997). Gene Therapy 4(5): 409-418.

Kren, B. T., Bandyopadhyay, P. and Steer, C. J. (1998). Nature Med. 4(3): 285-290.

Lee, R. J. and Huang, L. (1997). Crit. Rev. Ther. Drug Carrier Syst. 1997 14(2)(2): 173-206.

Nathan, A., Bolikal, D., Vyavahare, N., Zalipsky, S., Kohn, J. (1992) Macromolecules 25, 4476-4484.

Nathan, A., Zalipsky, S., Ertel, S. I., Agathos, S. N., Yarmush, M. L., Kohn, J. (1993). Bioconj. Chem. 4, 54-62.

Ogris, M., Brunner, S., Schuller, S., Kircheis, R. and Wagner, E. (1999). Gene Ther. 6(4): 595-605.

Plank, C., Mechtler, K., Szoka, F. C. and Wagner, E. (1996). Hum. Gene Ther. 7(12): 1437-1446.

Plank, C., Oberhauser, B., Mechtler, K., Koch, C., & Wagner, E. (1994) J. Biol. Chem. 269, 12918-12924.

Plank, C., Tang, M., Wolfe, A. and Szoka, F. C. (1999). Hum. Gene Ther. 10(2): 319-333.

Tang, M. X., Redemann, C. T. and Szoka, F. C. (1996). Bioconj. Chem. 7(6): 703-714.

Ulbrich, K., Strohalm, J., Kopecek, J. (1985). Macromol. Chem. 187, 1131-1144.

Wadhwa, M. S., Collard, W. T., Adami, R. C., McKenzie, D. L. and Rice, K. G. (1997). Bioconj. Chem. 8(1): 81-88.

Whaley, K. E. (1985). Churchill Livingstone, Edinburg, London, Melbourne and New York 1985.

Yoon, K., Colestrauss, A. and Kmiec, E. B. (1996). Proc. Natl. Acad. Sci. USA 93(5): 2071-2076.

Zalipsky, S., Gilon, C., Zilkha, A. (1984). J. Makromol. Sci.-Chem. A21, 839-845.

Zanta, M. A., Boussif, O., Adib, A., & Behr, J. P. (1997) Bioconj. Chem. 8, 839-844

Zhou, X. H. and Huang, L. (1994). Biochim. Et Biophys. Acta-Biomembr. 1189(2): 195-203.

**Patentansprüche**

**1.** Geladenes Copolymer der allgemeinen Formel I

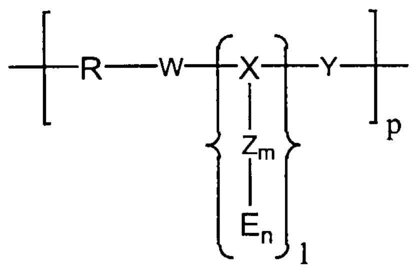

wobei R amphiphiles Polymer oder ein homo- oder hetero-bifunktionelles Derivat davon ist,
und worin X

i) eine Aminosäure oder ein Aminosäurederivat, ein Peptid oder ein Peptidderivat oder ein Spermin- oder Spermidinderivat ist; oder

ii) worin X

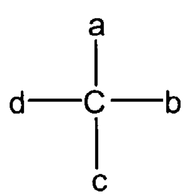

ist,
wobei
a H oder, gegebenenfalls halogen- oder dialkylamino-substituiertes, $C_1$-$C_6$-Alkyl bedeutet;
und wobei
b, c und d gleiches oder verschiedenes, gegebenfalls halogen- oder dialkylamino-substituiertes $C_1$-$C_6$-Alkylen bedeutet;
oder

iii) worin X

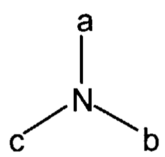

ist,

wobei

a H oder, gegebenenfalls halogen- oder dialkylamino-substituiertes, $C_1$-$C_6$-Alkyl bedeutet, und wobei

b und c gleiches oder verschiedenes, gegebenenfalls halogen- oder dialkylamino-substituiertes $C_1$-$C_6$-Alkylen bedeuten; oder

iv) worin X
eine substituierte aromatische Verbindung mit drei funktionellen Gruppierungen $W_1Y_1Z_1$ ist, wobei W, Y und Z die unten angegebenen Bedeutungen haben;
wobei
W, Y oder Z gleiche oder verschiedene Reste CO, NH, O oder S oder eine zur Reaktion mit SH, OH, NH oder $NH_2$ befähigte Linkergruppierung sind;
und wobei das Effektormolekül E ein kationisches oder anionisches Peptid oder Peptidderivat oder ein Spermin- oder Spermidinderivat oder ein Glykosaminoglycan oder ein nichtpeptidisches Oligo/Poly-kation oder -anion; worin
m und n unabhängig voneinander 0, 1 oder 2 sind; worin
p vorzugsweise 3 bis 20; und worin
l 1 bis 5, vorzugsweise 1 ist.

**2.** Copolymer nach Anspruch 1, worin das amphiphile Polymer ein Polyalkylenoxid ist.

**3.** Copolymer nach Anspruch 2, worin das amphiphile Polymer ein Polyalkylenglykol ist.

**4.** Copolymer nach einem der Ansprüche 1 bis 3, worin X oder E ein geladenes Peptid- oder Peptidderivat ist.

**5.** Copolymer nach einem der Ansprüche 1 bis 4, an das ein Ligand für eine höhere eukaryotische Zelle gekoppelt ist.

**6.** Komplex, enthaltend ein oder mehrere Nukleinsäuremoleküle und ein oder mehrere Copolymere nach einem der Ansprüche 1 bis 4.

**7.** Komplex, enthaltend ein oder mehrere Nukleinsäuremoleküle, kondensiert mit organischen Polykation- oder kationischen Lipidmolekülen, dadurch gekennzeichnet, daß er an seiner Oberfläche ein geladenes Copolymer der allgemeinen Formel I über ionische Wechselwirkung gebunden hat.

**8.** Komplex nach Anspruch 6 oder 7, enthaltend ein therapeutisch wirksames Nukleinsäuremolekül.

**9.** Pharmazeutische Zusammensetzung, enthaltend einen Komplex nach Anspruch 8.

**Fig. 1**

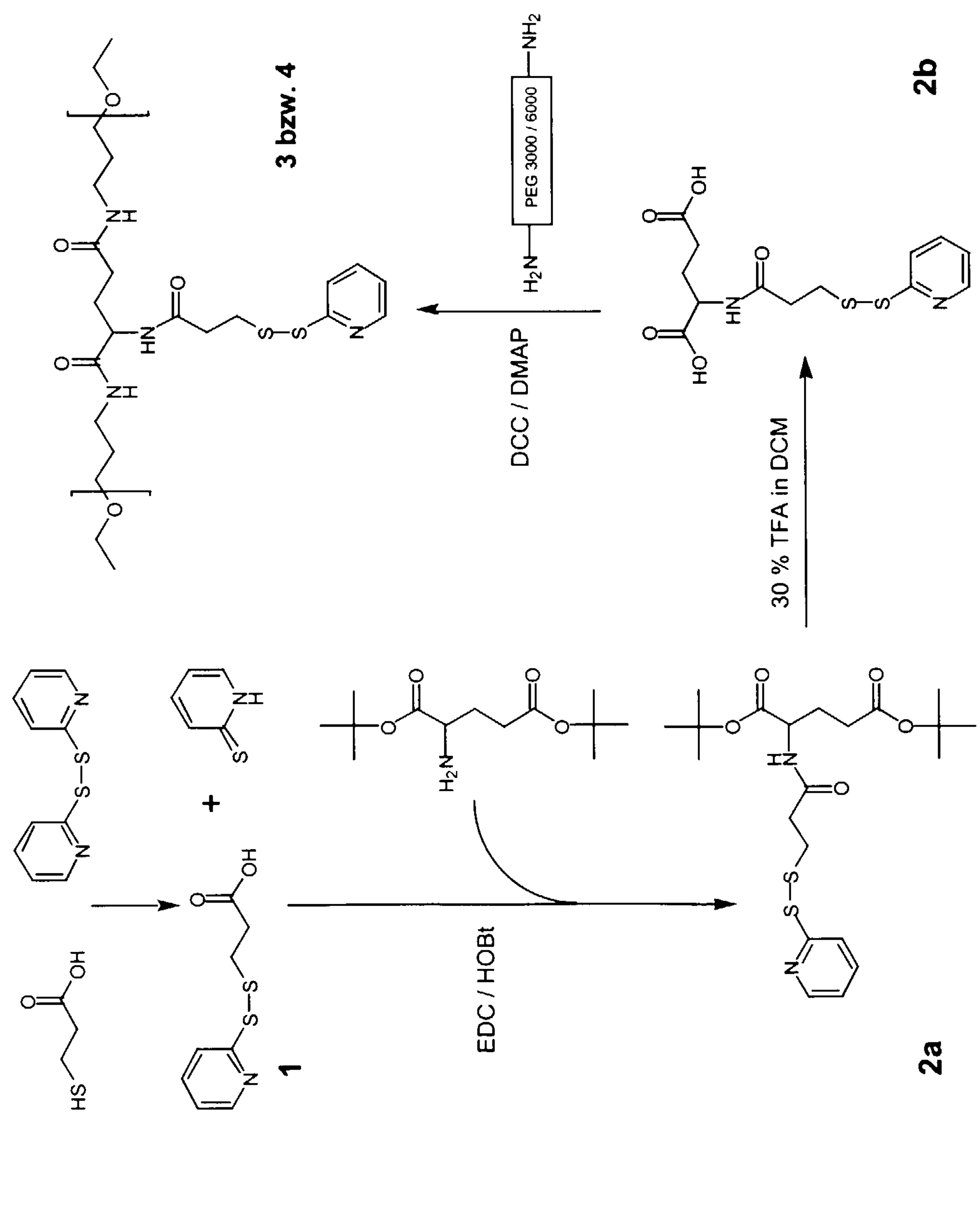


1

2a

2b

3 bzw. 4

**Fig. 2**

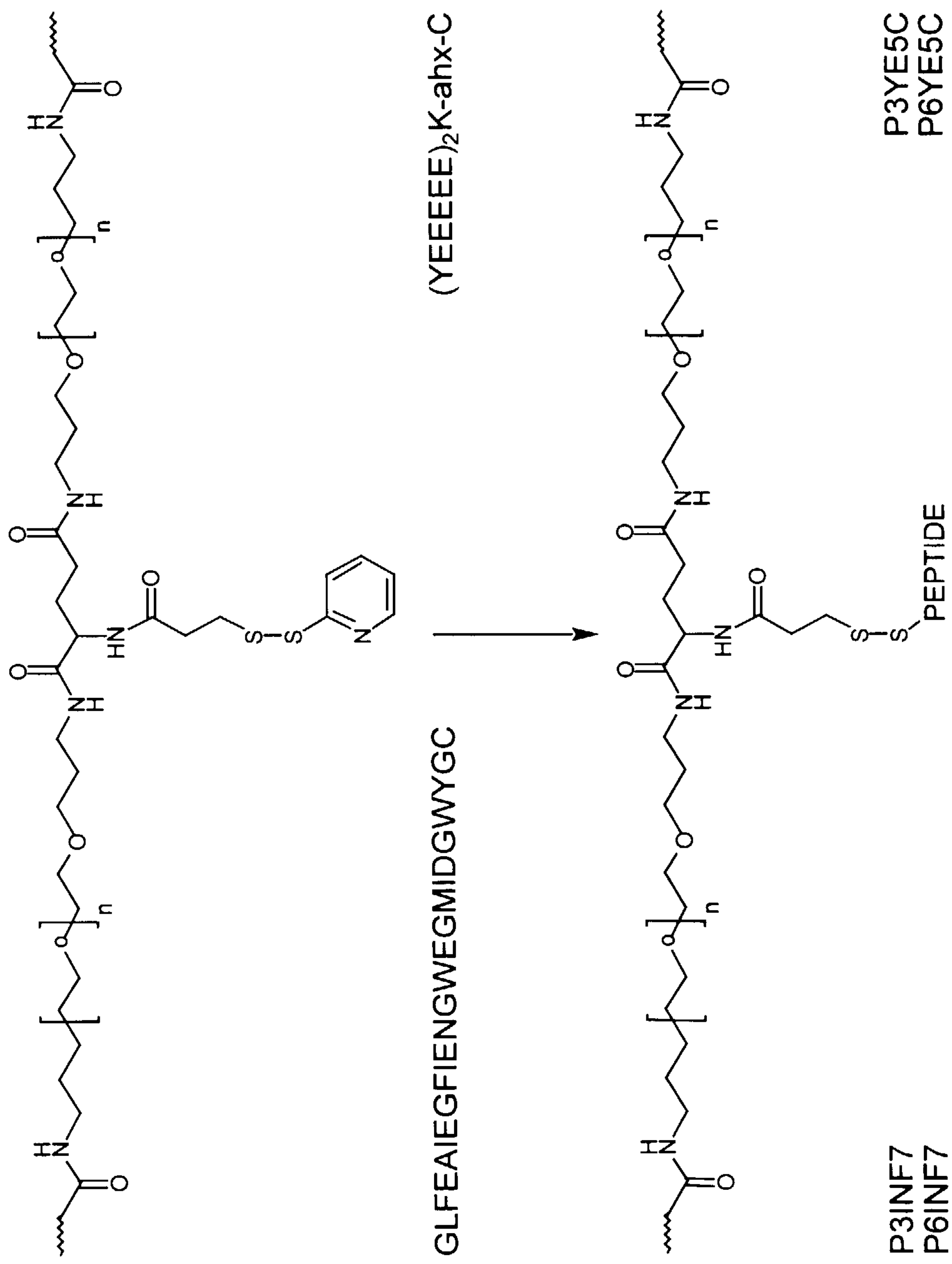
(YEEEEE)$_2$K-ahx-C
GLFEAIEGFIENGWEGMIDGWYGC
P3YE5C
P6YE5C
P3INF7
P6INF7

**Fig. 3**

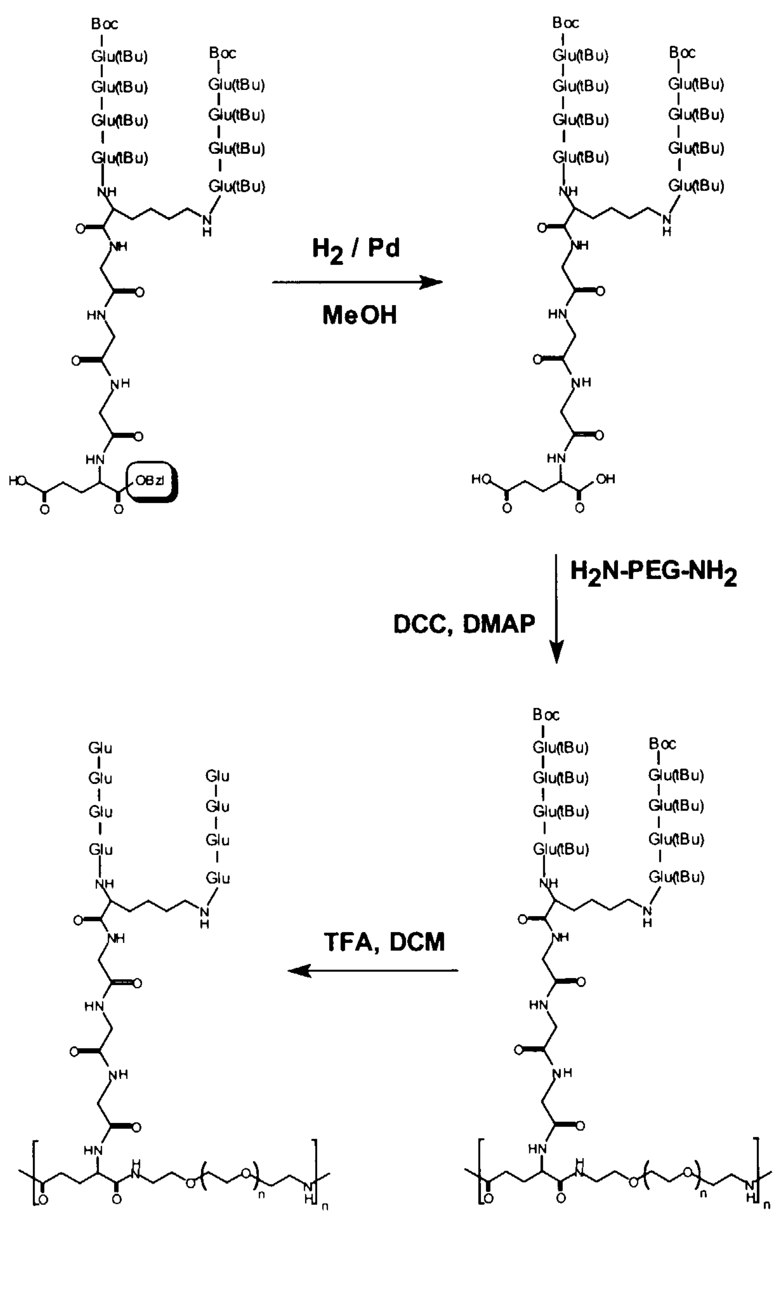

**Fig. 4/1**

**A**

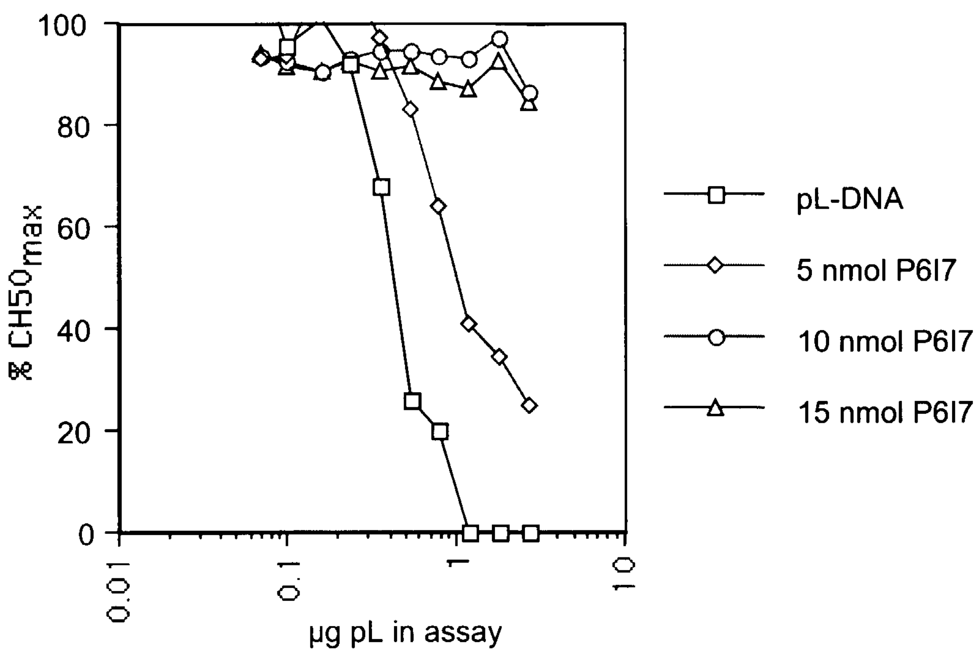
100
80
60
40
20
0
% CH50max
0.01
0.1
1
10
µg pL in assay
pL-DNA
5 nmol P6I7
10 nmol P6I7
15 nmol P6I7

**B**

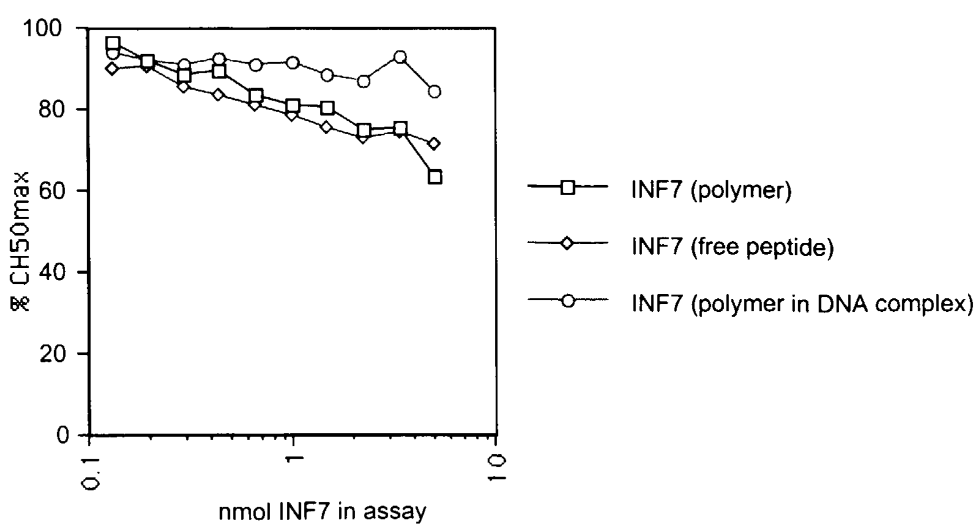
100
80
60
40
20
0
% CH50max
0.1
1
10
nmol INF7 in assay
INF7 (polymer)
INF7 (free peptide)
INF7 (polymer in DNA complex)

**Fig. 4/2**

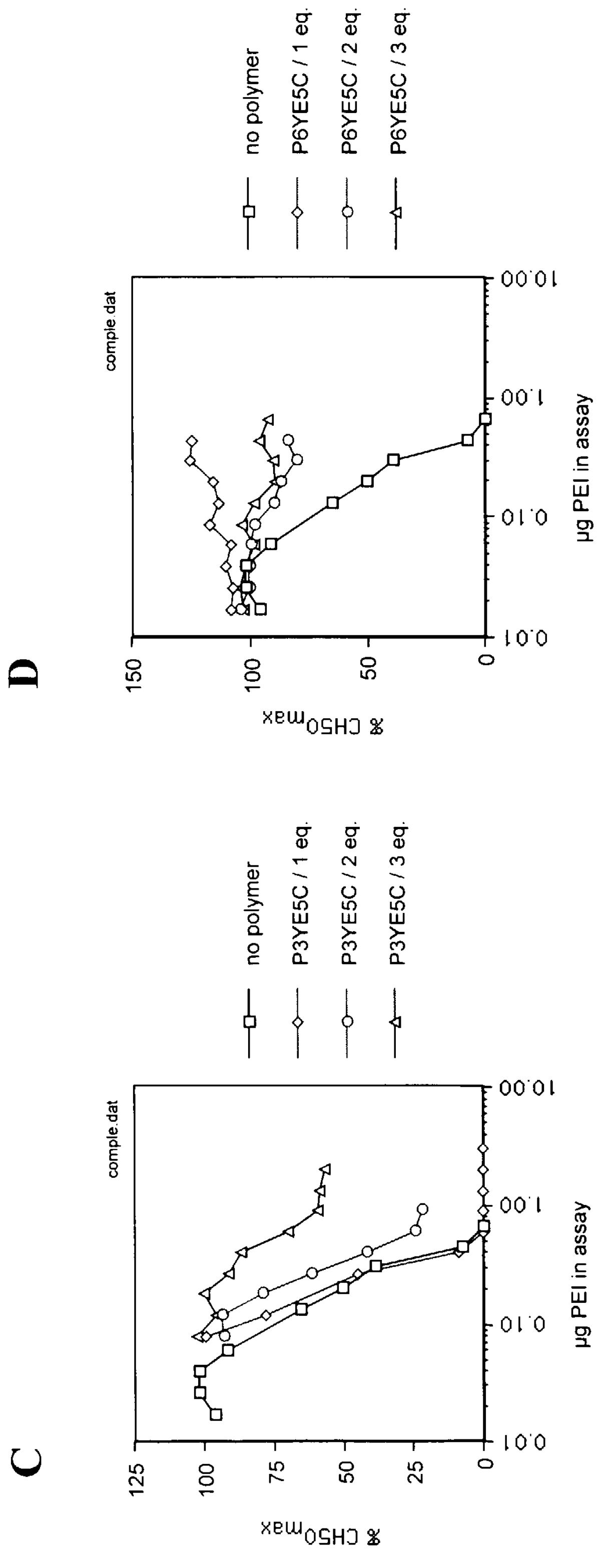
D
comple.dat
% CH50max
150
100
50
0
0.01
0.10
1.00
10.00
µg PEI in assay
no polymer
P6YE5C / 1 eq.
P6YE5C / 2 eq.
P6YE5C / 3 eq.
C
comple.dat
% CH50max
125
100
75
50
25
0
0.01
0.10
1.00
10.00
µg PEI in assay
no polymer
P3YE5C / 1 eq.
P3YE5C / 2 eq.
P3YE5C / 3 eq.

**Fig. 5**

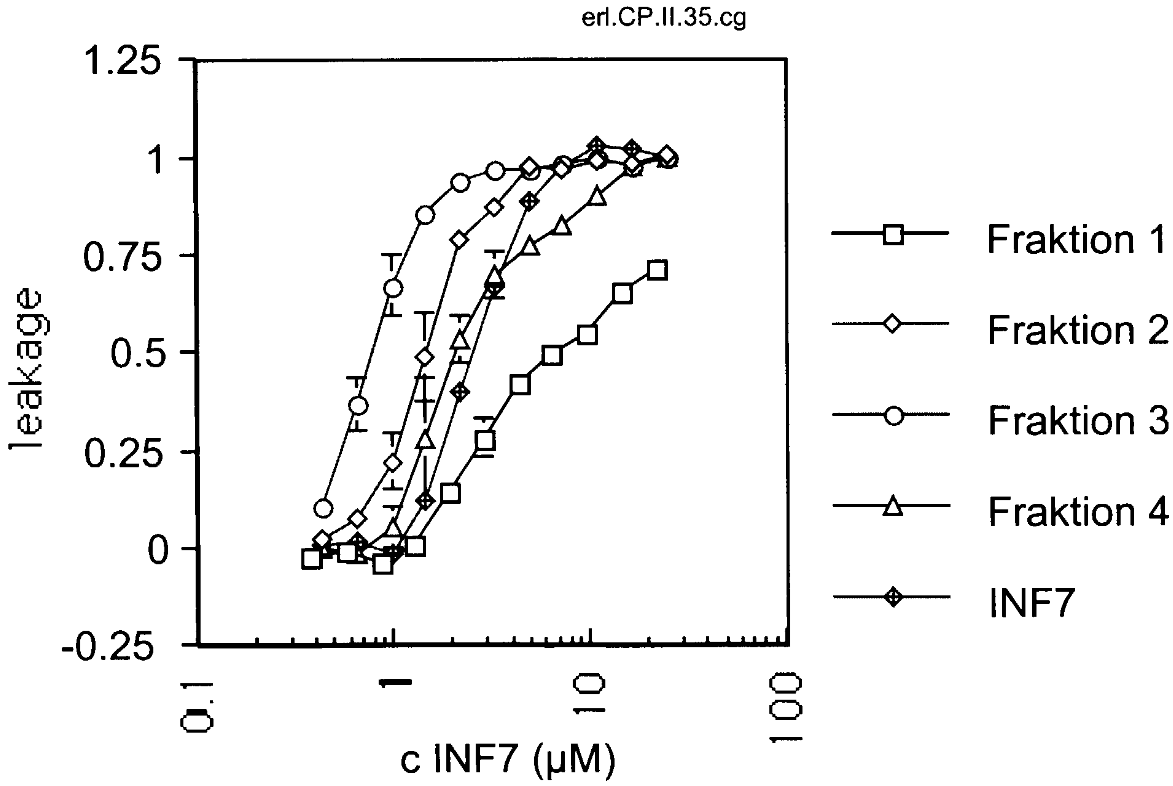
erl.CP.II.35.cg
1.25
1
0.75
0.5
0.25
0
-0.25
leakage
0.1
1
10
100
c INF7 (µM)
Fraktion 1
Fraktion 2
Fraktion 3
Fraktion 4
INF7

Fig. 6

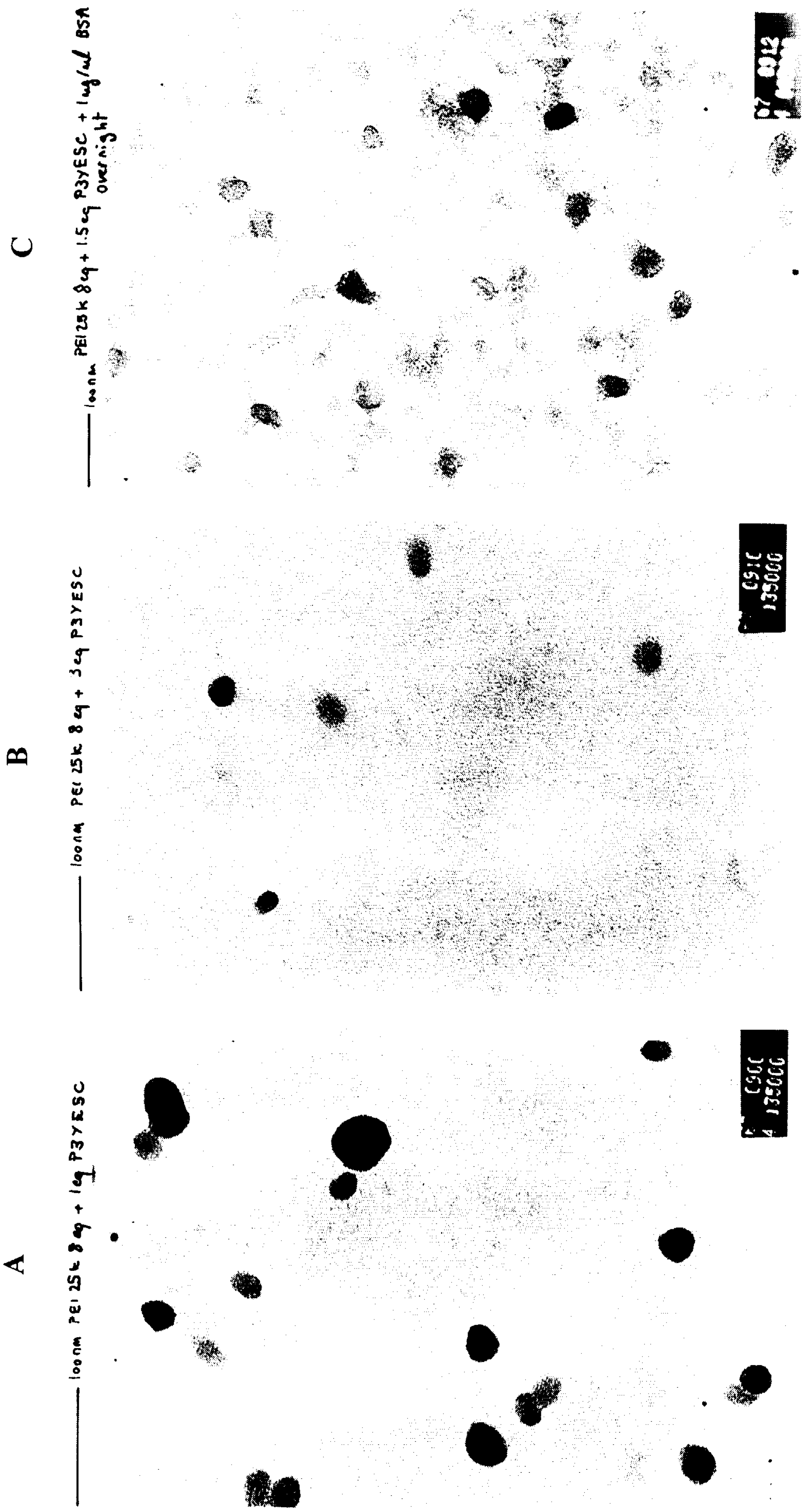
A
100nm PEI 25k 8eq + 1eq P3YESC
B
100nm PEI 25k 8eq + 3eq P3YESC
C
100nm PEI25k 8eq + 1.5eq P3YESC + 1ug/ul BSA overnight

**Fig. 7**

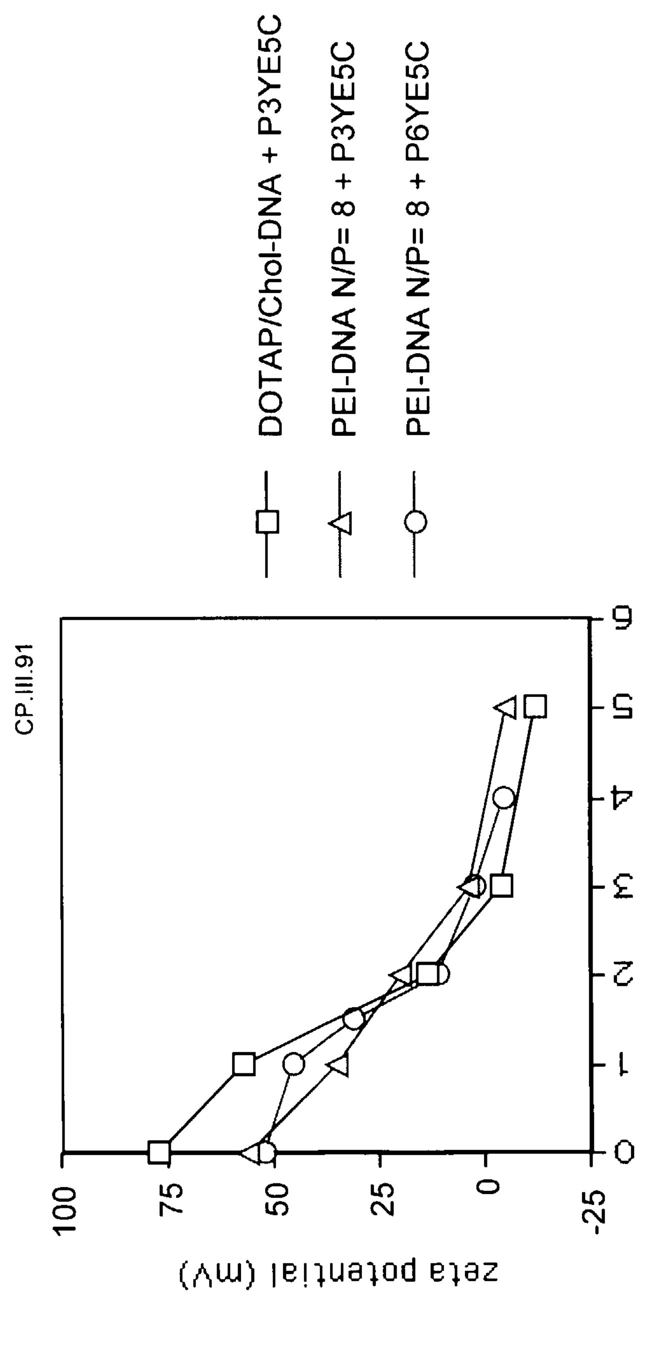
CP.III.91
zeta potential (mV)
100
75
50
25
0
-25
0
1
2
3
4
5
6
charge equivalents Polymer
DOTAP/Chol-DNA + P3YE5C
PEI-DNA N/P= 8 + P3YE5C
PEI-DNA N/P= 8 + P6YE5C

Fig. 8

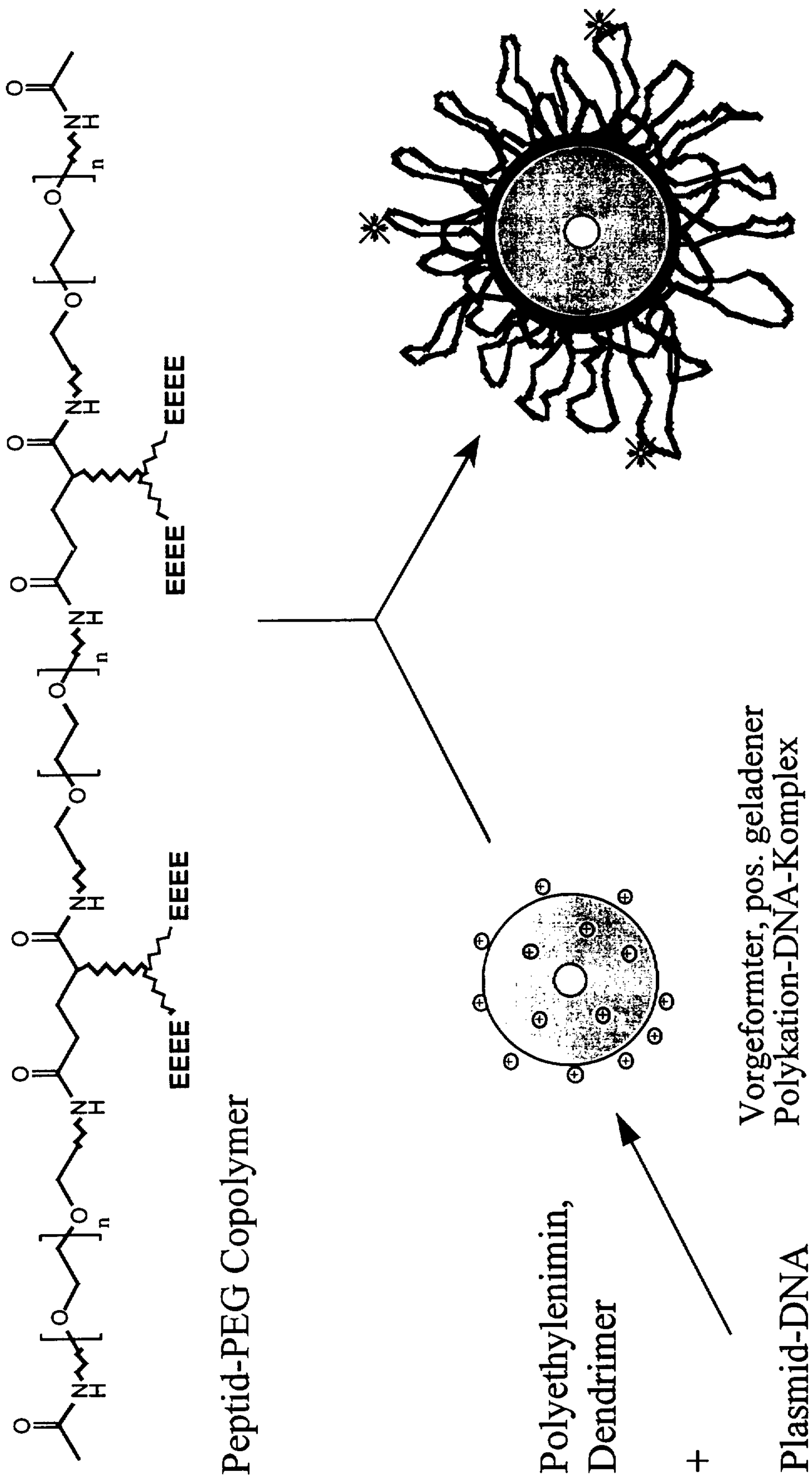
Peptid-PEG Copolymer
Polyethylenimin,
Dendrimer
+
Plasmid-DNA
Vorgeformter, pos. geladener
Polykation-DNA-Komplex

Fig. 9

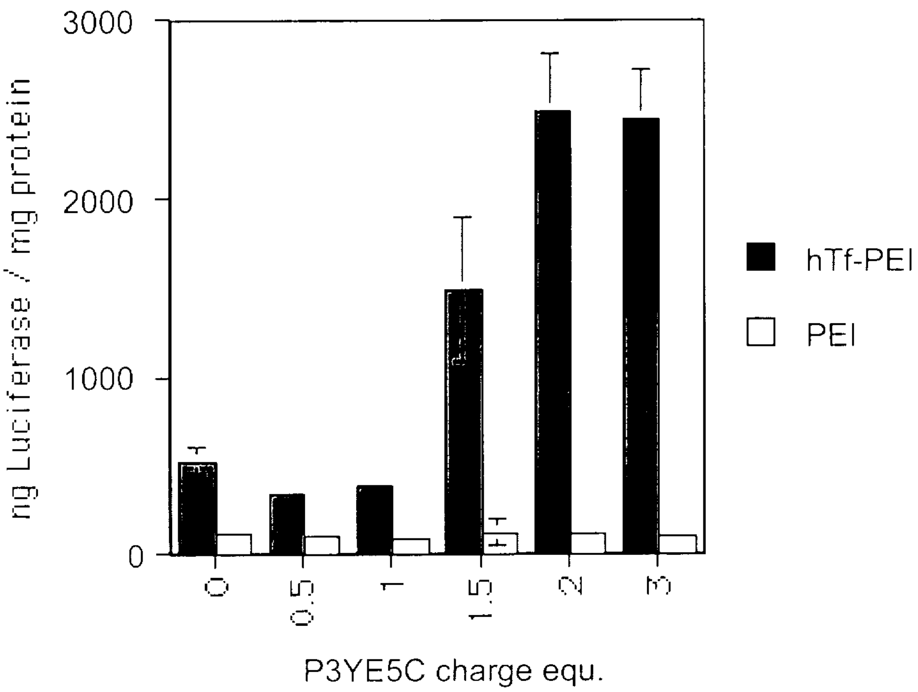
3000
2000
1000
0
ng Luciferase / mg protein
0
0.5
1
1.5
2
3
P3YE5C charge equ.
hTf-PEI
PEI

Fig. 10

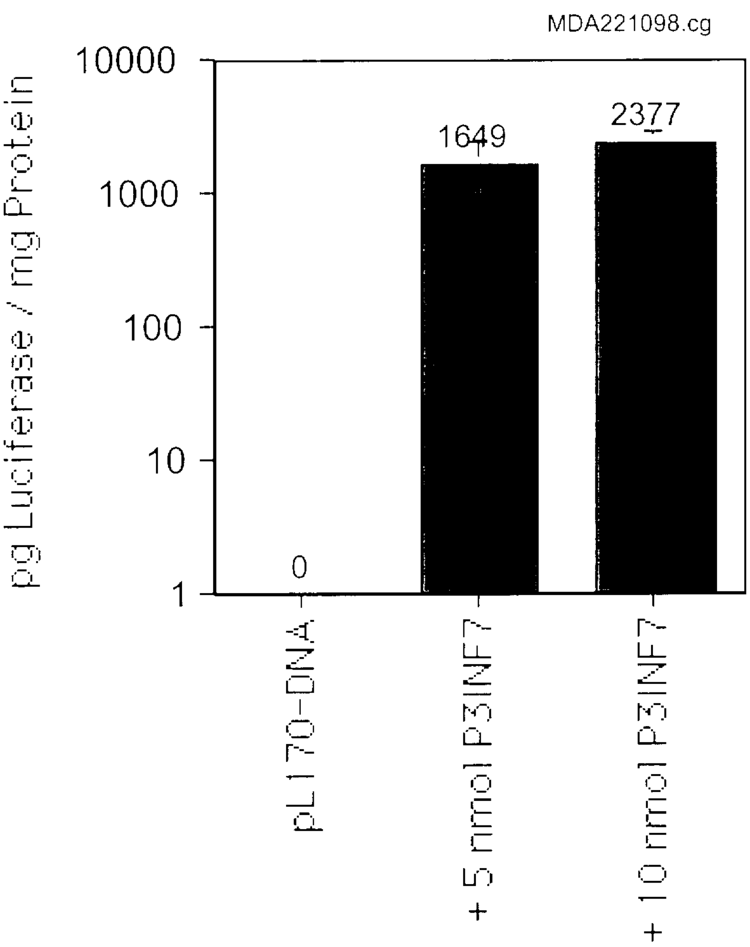
MDA221098.cg
pg Luciferase / mg Protein
10000
1000
100
10
1
0
1649
2377
pL170-DNA
+ 5 nmol P3INF7
+ 10 nmol P3INF7

**Fig. 11**

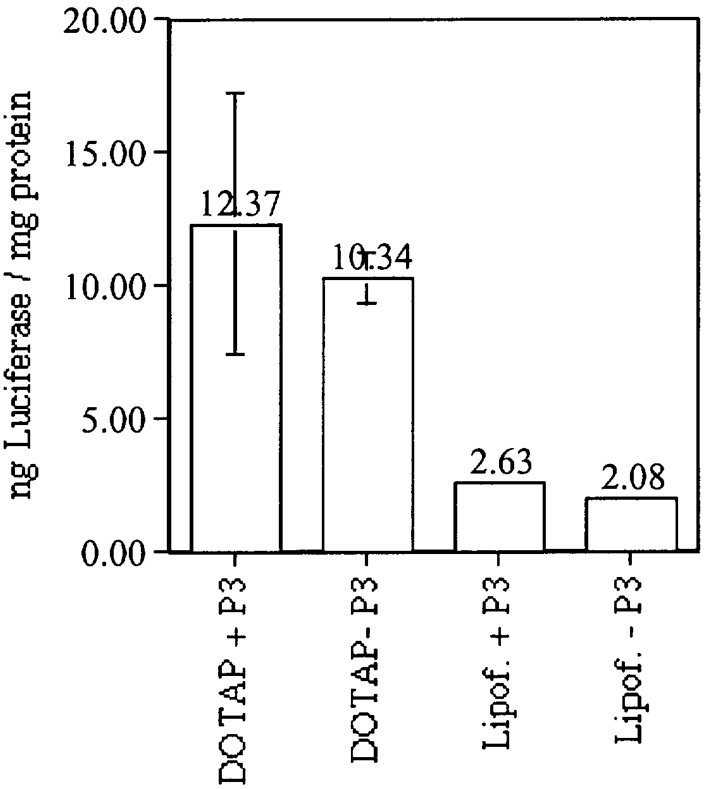
20.00
15.00
10.00
5.00
0.00
ng Luciferase / mg protein
12.37
10.34
2.63
2.08
DOTAP + P3
DOTAP- P3
Lipof. + P3
Lipof. - P3

**Fig. 12**

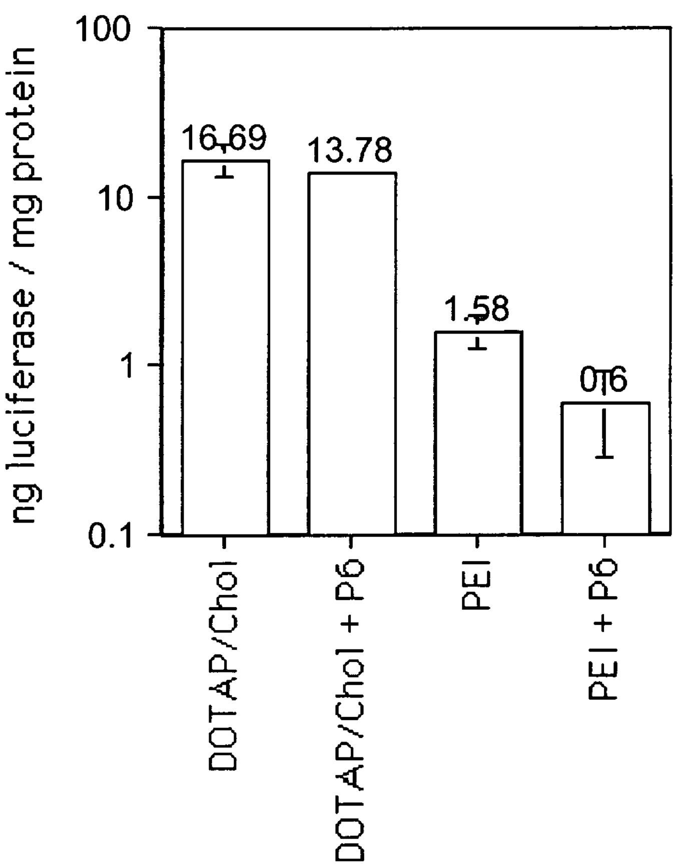
100
10
1
0.1
ng luciferase / mg protein
16.69
13.78
1.58
0.6
DOTAP/Chol
DOTAP/Chol + P6
PEI
PEI + P6

# Fig. 13

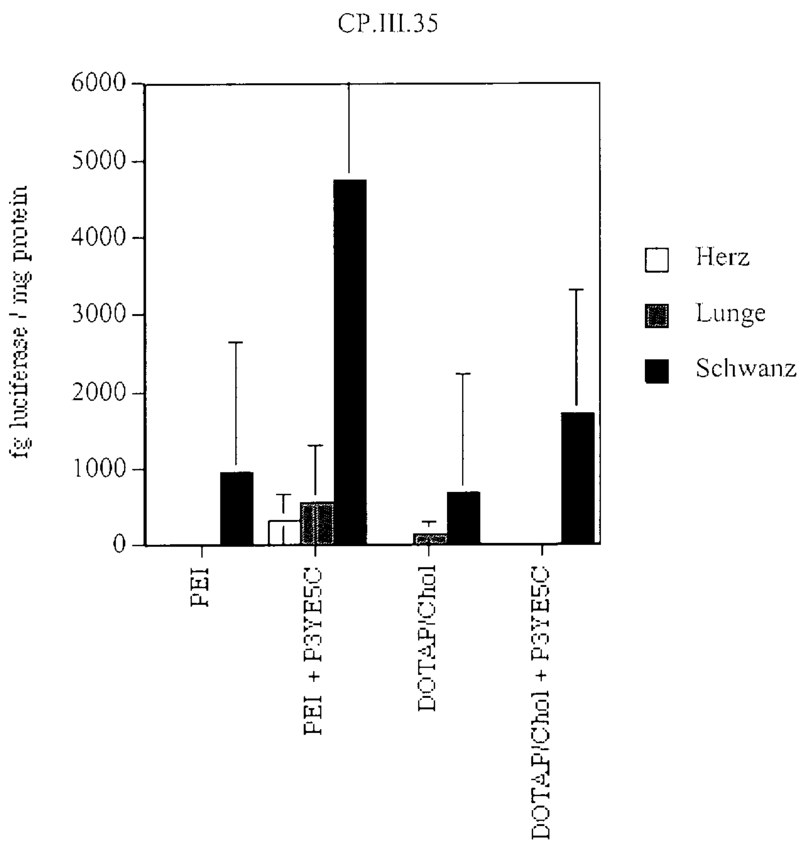
CP.III.35
fg luciferase / mg protein
6000
5000
4000
3000
2000
1000
0
PEI
PEI + P3YE5C
DOTAP/Chol
DOTAP/Chol + P3YE5C
Herz
Lunge
Schwanz

Europäisches Patentamt

# EUROPÄISCHER RECHERCHENBERICHT

Nummer der Anmeldung
EP 99 11 2260

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | WO 98 19710 A (SCHACHT ETIENNE HONORE ;ULBRICH KAREL (CZ); SEYMOUR LEONARD CHARLE) 14. Mai 1998 (1998-05-14)<br>* Abbildung 4 *<br>* Ansprüche 1,10,11 *<br>--- | 1-9 | C08G65/329<br>C08G65/333<br>A61K48/00<br>C12N15/87<br>A61K47/48 |
| X | WO 96 21036 A (VIAGENE INC)<br>11. Juli 1996 (1996-07-11)<br>* Ansprüche 1,3,4 *<br>--- | 1-9 | |
| X | WO 97 25067 A (DAVIS STANLEY STEWART ;UNIV NOTTINGHAM (GB); GARNETT MARTIN CHARLE)<br>17. Juli 1997 (1997-07-17)<br>* Ansprüche 1,6-8 *<br>* Seite 3, Zeile 24 - Zeile 25 *<br>--- | 1-9 | |
| X | PATENT ABSTRACTS OF JAPAN<br>vol. 1998, no. 06,<br>30. April 1998 (1998-04-30)<br>& JP 10 028583 A (HISAMITSU PHARMACEUT CO INC), 3. Februar 1998 (1998-02-03)<br>* Zusammenfassung *<br>--- | 1-6,8,9 | RECHERCHIERTE SACHGEBIETE (Int.Cl.7) |
| X | WO 94 09056 A (STERLING WINTHROP INC)<br>28. April 1994 (1994-04-28)<br>* Ansprüche 1,4,9 *<br>* Seite 4, Zeile 20 - Zeile 21 *<br>* Seite 9, Zeile 10 - Zeile 30 *<br>--- | 1-5 | C08G<br>A61K<br>C12N |
| X,D | US 5 455 027 A (ZALIPSKY SAMUEL ET AL)<br>3. Oktober 1995 (1995-10-03)<br>* Spalte 4, Zeile 15-20 *<br>* Spalte 27, Zeile 16 *<br>----- | 1-5 | |

Der vorliegende Recherchenbericht wurde für alle Patentansprüche erstellt

| Recherchenort | Abschlußdatum der Recherche | Prüfer |
|---|---|---|
| DEN HAAG | 9. Dezember 1999 | O'Sullivan, T |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 99 11 2260

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-12-1999

| Im Recherchenbericht angeführtes Patentdokument | Datum der Veröffentlichung | Mitglied(er) der Patentfamilie | Datum der Veröffentlichung |
|---|---|---|---|
| WO 9819710 A | 14-05-1998 | AU 4873997 A | 29-05-1998 |
| | | EP 0941123 A | 15-09-1999 |
| WO 9621036 A | 11-07-1996 | AU 4690596 A | 24-07-1996 |
| WO 9725067 A | 17-07-1997 | AU 1386697 A | 01-08-1997 |
| | | CA 2241830 A | 17-07-1997 |
| | | EP 0871491 A | 21-10-1998 |
| | | GB 2324534 A | 28-10-1998 |
| | | NO 982894 A | 22-06-1998 |
| JP 10028583 A | 03-02-1998 | KEINE | |
| WO 9409056 A | 28-04-1994 | AU 5323794 A | 09-05-1994 |
| | | CA 2146990 A | 28-04-1994 |
| | | DE 69312951 D | 11-09-1997 |
| | | DE 69312951 T | 11-12-1997 |
| | | EP 0664819 A | 02-08-1995 |
| | | ES 2105332 T | 16-10-1997 |
| | | HK 1001498 A | 19-06-1998 |
| | | JP 8502528 T | 19-03-1996 |
| | | US 5583206 A | 10-12-1996 |
| US 5455027 A | 03-10-1995 | US 5219564 A | 15-06-1993 |
| | | US 5720950 A | 24-02-1998 |
| | | US 5660822 A | 26-08-1997 |
| | | US 5372807 A | 13-12-1994 |
| | | WO 9200748 A | 23-01-1993 |

EPO FORM P0461

Für nähere Einzelheiten zu diesem Anhang : siehe Amtsblatt des Europäischen Patentamts, Nr.12/82